(19) Europäisches Patentamt
European Patent Office
Office européen des brevets

(11) **EP 4 580 244 A1**

(12) **EUROPEAN PATENT APPLICATION**
published in accordance with Art. 153(4) EPC

(43) Date of publication:
**02.07.2025 Bulletin 2025/27**

(21) Application number: **22963837.4**

(22) Date of filing: **01.11.2022**

(51) International Patent Classification (IPC):
**H04W 24/08** $^{(2009.01)}$    **H04W 24/02** $^{(2009.01)}$
**H04W 4/50** $^{(2018.01)}$

(52) Cooperative Patent Classification (CPC):
**H04W 4/50; H04W 24/02; H04W 24/08**

(86) International application number:
**PCT/CN2022/129033**

(87) International publication number:
**WO 2024/092519 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Huawei Technologies Co., Ltd.**
**Shenzhen, Guangdong 518129 (CN)**

(72) Inventors:
- **ZHENG, Yaoyao**
  **Shenzhen, Guangdong 518129 (CN)**
- **ZENG, Kun**
  **Shenzhen, Guangdong 518129 (CN)**
- **LIU, Qiao**
  **Shenzhen, Guangdong 518129 (CN)**
- **WANG, Guangjian**
  **Shenzhen, Guangdong 518129 (CN)**

(74) Representative: **Goddar, Heinz J.**
**Boehmert & Boehmert**
**Anwaltspartnerschaft mbB**
**Pettenkoferstrasse 22**
**80336 München (DE)**

(54) **GAS CONCENTRATION DETERMINATION METHOD AND RELATED APPARATUS**

(57) A method for determining a gas concentration and a related apparatus are provided, and the method is applicable to a first communication device. The method includes: sending a first communication signal to a second communication device; receiving a first echo signal of the first communication signal, where a frequency of the first communication signal is $f_1$, and $f_1$ is used for a first gas; determining a first concentration of the first gas based on transmit power of the first communication signal and receive power of the first echo signal; and determining, based on the first concentration, whether to send a second communication signal to the second communication device. In the method, a wireless communication system is used to estimate a gas concentration, thereby reducing costs of estimating the gas concentration.

A first communication device sends a first communication signal to a second communication device — S201

The first communication device receives a first echo signal of the first communication signal — S202

The first communication device determines a first concentration of a first gas based on transmit power of the first communication signal and receive power of the first echo signal — S203

The first communication device determines, based on the first concentration, whether to send a second communication signal to the second communication device — S204

FIG. 2

**EP 4 580 244 A1**

## Description

### TECHNICAL FIELD

This application relates to the field of communication technologies, and in particular, to a method for determining a gas concentration and a related apparatus.

### BACKGROUND

Gas detection may be applied to multiple scenarios. For example, in industrial scenarios, a production line progress can be effectively monitored by detecting a gas that is used as a primary (secondary) product; in environmental monitoring, air quality can be monitored in real time by detecting a concentration of a specific gas in an atmosphere, which facilitates a supervision department to targetedly carry out an air pollution prevention and control solution; and in agricultural and animal husbandry scenarios, life cycles or physiological statuses of crops and livestock can be monitored by detecting gases released by the crops and livestock. Currently, gas detection requires a dedicated gas detection device. However, the dedicated gas detection device is large in size and is expensive, which is inconducive to large-scale deployment.

### SUMMARY

This application provides a method for determining a gas concentration and a related apparatus, to reduce costs of estimating a gas concentration by estimating the gas concentration by a wireless communication system.

According to a first aspect, this application provides a method for determining a gas concentration. The method is applied to a first communication device, and the method includes:

sending a first communication signal to a second communication device;
receiving a first echo signal of the first communication signal, where a frequency of the first communication signal is $f_1$, and $f_1$ is used for a first gas;
determining a first concentration of the first gas based on transmit power of the first communication signal and receive power of the first echo signal; and
determining, based on the first concentration, whether to send a second communication signal to the second communication device.

According to the first aspect, the first communication device sends the first communication signal to the second communication device, and the first communication device receives the echo signal (namely, the first echo signal) of the first communication signal, so that the first communication device may determine the first concentration of the first gas based on the transmit power of the first communication signal and the receive power of the first echo signal, in other words, a wireless communication system may be used to detect the gas around the communication link, to estimate the gas concentration. Further, whether to send the second communication signal may be further determined based on the first concentration. If the second communication signal is determined to be sent, transmit power of the second communication signal and receive power of the second echo signal may be combined with the transmit power of the first communication signal and the receive power of the first echo signal, to estimate a more accurate gas concentration. If the second communication signal is determined not to be sent, energy saving of the first communication device is facilitated.

In a possible implementation, the determining, based on the first concentration, whether to send a second communication signal to the second communication device includes:
determining, based on one of the following, to send the second communication signal to the second communication device:

when the first concentration is greater than or equal to a first concentration threshold, the second communication signal is determined to be sent to the second communication device; or
when the first concentration is less than a first concentration threshold and the second communication signal is not sent to the second communication device within preset duration, the second communication signal is determined to be sent to the second communication device.

In a possible implementation, the determining, based on the first concentration, whether to send a second communication signal to the second communication device includes:
determining, based on the following condition, not to send the second communication signal to the second communication device:

when the first concentration is less than a first concentration threshold, the second communication signal is determined not to be sent to the second communication device.

In a possible implementation, after the second communication signal is determined, based on the first concentration, to be sent to the second communication device, the method further includes:

sending the first communication signal and the second communication signal to the second communication device;
receiving the first echo signal of the first communication signal and a second echo signal of the second communication signal, where a frequency of the second communication signal is $f_2$, $f_2$ is used for the first gas, and an absorption coefficient of the first gas at the frequency of $f_1$ is greater than an absorption coefficient of the first gas at the frequency of $f_2$; and
determining a second concentration of the first gas based on the transmit power of the first communication signal, the receive power of the first echo signal, transmit power of the second communication signal, and receive power of the second echo signal.

In a possible implementation, after the sending the first communication signal and the second communication signal to the second communication device, the method further includes:

when the first concentration is less than the first concentration threshold, stopping sending the second communication signal to the second communication device or reducing a quantity of sending times of sending the second communication signal to the second communication device within unit time; or
when the second concentration is less than the first concentration threshold, stopping sending the second communication signal to the second communication device or reducing a quantity of sending times of sending the second communication signal to the second communication device within unit time.

In a possible implementation, after the sending the first communication signal and the second communication signal to the second communication device, the method further includes:
when a concentration difference between the first concentration and the second concentration is less than or equal to a second concentration threshold, stopping sending the second communication signal to the second communication device or reducing a quantity of sending times of sending the second communication signal to the second communication device within unit time.

In a possible implementation, a quantity of sending times of the first communication signal within the unit time is greater than or equal to the quantity of sending times of the second communication signal within the unit time.

In a possible implementation, the determining a first concentration of the first gas based on transmit power of the first communication signal and receive power of the first echo signal includes:
determining the first concentration of the first gas based on the transmit power of the first communication signal, the receive power of the first echo signal, a distance between the first communication device and the second communication device, and the absorption coefficient of the first gas at the frequency of $f_1$.

In a possible implementation, the first concentration satisfies:

$$M_A = \frac{1}{2L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

In a possible implementation, the determining a second concentration of the first gas based on the transmit power of the first communication signal, the receive power of the first echo signal, transmit power of the second communication signal, and receive power of the second echo signal includes:
determining the second concentration of the first gas on a communication link between the second communication device and the second communication device based on the transmit power of the first communication signal, the receive power of the first echo signal, the transmit power of the second communication signal, the receive power of the second echo signal, a distance between the first communication device and the second communication device, the absorption coefficient of the first gas at the frequency of $f_1$, and the absorption coefficient of the first gas at the frequency of $f_2$.

In a possible implementation, the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first echo signal, $P_{B0}(f_1)$ represents the transmit power of the second communication signal, and $P_A(f_1)$ represents the receive power of the second echo signal.

In a possible implementation, both $f_1$ and $f_2$ belong to a licensed frequency band, or $f_1$ belongs to a licensed frequency band, and $f_2$ belongs to an unlicensed frequency band.

According to a second aspect, this application provides a method for determining a gas concentration. The method is applied to a first communication device, and the method includes:

sending a first communication signal to a second communication device, where a frequency of the first communication signal is $f_1$, $f_1$ is used for a first gas, and transmit power and receive power of the first communication signal are used to determine a first concentration of the first gas; and

determining, based on first indication information from the second communication device, whether to send a second communication signal to the second communication device, where a frequency of the second communication signal is $f_2$, $f_2$ is used for the first gas, and an absorption coefficient of the first gas at the frequency of $f_1$ is greater than an absorption coefficient of the first gas at the frequency of $f_2$.

According to the second aspect, the first communication device sends the first communication signal to the second communication device, and correspondingly, the second communication device receives the first communication signal from the first communication device, so that the second communication device may determine the first concentration of the first gas based on the transmit power and the receive power of the first communication signal, in other words, a wireless communication system may be used to detect the gas around the communication link between the first communication device and the second communication device, to estimate the gas concentration. Further, the first communication device may further determine, based on the first indication information from the second communication device, whether to send the second communication signal. If the second communication signal is determined to be sent, the second communication device may combine transmit power and receive power of the second communication signal with the transmit power and the receive power of the first communication signal, to estimate a more accurate gas concentration. If the second communication signal is determined not to be sent, energy saving of the first communication device is facilitated.

In a possible implementation, the first indication information indicates the first communication device to send the second communication signal to the second communication device.

In a possible implementation, the first indication information includes the first concentration.

In a possible implementation, after the second communication signal is determined to be sent to the second communication device based on the first indication information from the second communication device, the method further includes:

sending the first communication signal and the second communication signal to the second communication device.

In a possible implementation, a quantity of sending times of the first communication signal within unit time is greater than or equal to a quantity of sending times of the second communication signal within the unit time.

In a possible implementation, after the sending the first communication signal and the second communication signal to the second communication device, the method further includes:

determining, based on second indication information from the second communication device, to stop sending the second communication signal to the second communication device or reduce the quantity of sending times of sending the second communication signal to the second communication device within the unit time.

In a possible implementation, the second indication information indicates to stop sending the second communication signal to the second communication device or reduce the quantity of sending times of sending the second communication signal to the second communication device within the unit time.

In a possible implementation, the second indication information includes the first concentration and/or a second concentration of the first gas, and the second concentration is determined based on the transmit power and the receive power of the first communication signal and transmit power and receive power of the second communication signal.

In a possible implementation, both $f_1$ and $f_2$ belong to a licensed frequency band, or $f_1$ belongs to a licensed frequency band, and $f_2$ belongs to an unlicensed frequency band.

According to a third aspect, this application provides a method for determining a gas concentration. The method is applied to a second communication device, and the method includes:

receiving a first communication signal from a first communication device, where a frequency of the first communication signal is $f_1$, and $f_1$ is used for a first gas;

determining a first concentration of the first gas based on receive power and transmit power of the first communication signal; and

determining, based on the first concentration, whether to send first indication information to the first communication device, where the first indication information is used to determine that the first communication device sends a second communication signal to the second communication device; or

sending first indication information to the first communication device, where the first indication information is used to determine whether the first communication device sends a second communication signal to the second communication device, and the first indication information includes the first concentration.

In a possible implementation, the determining, based on the first concentration, whether to send first indication information to the first communication device includes:

determining, based on one of the following, to send the first indication information to the first communication device:

when the first concentration is greater than or equal to a first concentration threshold, the first indication information is determined to be sent to the first communication device; or

when the first concentration is less than a first concentration threshold, and the first indication information is not sent to the first communication device within preset duration, the first indication information is determined to be sent to the first communication device.

In a possible implementation, the determining, based on the first concentration, whether to send first indication information to the first communication device includes:

determining, based on the following condition, not to send the first indication information to the first communication device:

when the first concentration is less than a first concentration threshold, the first indication information is determined not to be sent to the first communication device.

In a possible implementation, the first indication information is used to determine that the first communication device sends the second communication signal to the second communication device; and

after the first indication information is determined, based on the first concentration, to be sent to the second communication device, the method further includes:

receiving the first communication signal and the second communication signal from the first communication device; and

determining a second concentration of the first gas based on the transmit power and the receive power of the first communication signal and transmit power and receive power of the second communication signal.

In a possible implementation, after the receiving the first communication signal and the second communication signal from the first communication device, the method further includes:

when the first concentration is less than the first concentration threshold, sending second indication information to the first communication device, where the second indication information indicates the first communication device to stop sending the second communication signal to the second communication device or reduce a quantity of sending times of sending the second communication signal to the second communication device within unit time; or

when the second concentration is less than the first concentration threshold, sending second indication information to the first communication device, where the second indication information indicates the first communication device to stop sending the second communication signal to the second communication device or reduce a quantity of sending times of sending the second communication signal to the second communication device within unit time.

In a possible implementation, after the receiving the first communication signal and the second communication signal from the first communication device, the method further includes:

when a concentration difference between the first concentration and the second concentration is less than or equal to a second concentration threshold, sending second indication information to the first communication device, where the second indication information indicates the first communication device to stop sending the second communication signal to the second communication device or reduce a quantity of sending times of sending the second communication signal to the second communication device within unit time.

In a possible implementation, after the receiving the first communication signal and the second communication signal from the first communication device, the method further includes:

sending second indication information to the first communication device, where the second indication information includes the first concentration and/or the second concentration.

In a possible implementation, the determining a first concentration of the first gas based on receive power and transmit power of the first communication signal includes:

determining the first concentration of the first gas based on the transmit power of the first communication signal, the receive power of the first communication signal, a distance between the second communication device and the first communication device, and an absorption coefficient of the first gas at the frequency of $f_1$.

In a possible implementation, the first concentration satisfies:

$$M_A = \frac{1}{L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the second communication device and the first communication device, $P_{A0}(f_1)$ represents the transmit power of the first communication signal, and $P_A(f_1)$ represents the receive power of the first communication signal.

In a possible implementation, the determining a second concentration of the first gas based on the transmit power and the receive power of the first communication signal and transmit power and receive power of the second communication signal includes:

determining the second concentration of the first gas on a communication link between the first communication device and the first communication device based on the transmit power of the first communication signal, the receive power of the first communication signal, the transmit power of the second communication signal, the receive power of the second communication signal, a distance between the second communication device and the first communication device, an absorption coefficient of the first gas at the frequency of $f_1$, and an absorption coefficient of the first gas at a frequency of $f_2$.

In a possible implementation, the second concentration satisfies:

$$M_{AB} = \frac{1}{\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the second communication device and the first communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

In a possible implementation, both $f_1$ and $f_2$ belong to a licensed frequency band, or $f_1$ belongs to a licensed frequency band, and $f_2$ belongs to an unlicensed frequency band.

According to a fourth aspect, this application provides an apparatus for determining a gas concentration. The apparatus is a first communication device, and the apparatus includes:

a transceiver unit, configured to send a first communication signal to a second communication device, where the transceiver unit is configured to receive a first echo signal of the first communication signal, where a frequency of the first communication signal is $f_1$, and $f_1$ is used for a first gas; and
a processing unit, configured to determine a first concentration of the first gas based on transmit power of the first communication signal and receive power of the first echo signal, where
the processing unit is configured to determine, based on the first concentration, whether to send a second communication signal to the second communication device.

In a possible implementation, when performing the determining, based on the first concentration, whether to send the second communication signal to the second communication device, the processing unit is configured to:
determine, based on one of the following, to send the second communication signal to the second communication device:

when the first concentration is greater than or equal to a first concentration threshold, the second communication signal is determined to be sent to the second communication device; or
when the first concentration is less than a first concentration threshold and the second communication signal is not sent to the second communication device within preset duration, the second communication signal is determined to be sent to the second communication device.

In a possible implementation, when performing the determining, based on the first concentration, whether to send the second communication signal to the second communication device, the processing unit is configured to:
determine, based on the following condition, not to send the second communication signal to the second communication device:
when the first concentration is less than a first concentration threshold, the second communication signal is determined not to be sent to the second communication device.

In a possible implementation, the transceiver unit is configured to send the first communication signal and the second communication signal to the second communication device;

the transceiver unit is configured to receive the first echo signal of the first communication signal and a second echo signal of the second communication signal, where a frequency of the second communication signal is $f_2$, $f_2$ is used for the first gas, and an absorption coefficient of the first gas at the frequency of $f_1$ is greater than an absorption coefficient of the first gas at the frequency of $f_2$; and
the processing unit is configured to determine a second concentration of the first gas based on the transmit power of the first communication signal, the receive power of the first echo signal, transmit power of the second communication signal, and receive power of the second echo signal.

In a possible implementation, the processing unit is further configured to:

when the first concentration is less than the first concentration threshold, stop sending the second communication signal to the second communication device or reduce a quantity of sending times of sending the second communication signal to the second communication device within unit time; or
when the second concentration is less than the first concentration threshold, stop sending the second communication signal to the second communication device or reduce a quantity of sending times of sending the second communication signal to the second communication device within unit time.

In a possible implementation, the processing unit is further configured to:
when a concentration difference between the first concentration and the second concentration is less than or equal to a second concentration threshold, stop sending the second communication signal to the second communication device or reduce a quantity of sending times of sending the second communication signal to the second communication device within unit time.

In a possible implementation, a quantity of sending times of the first communication signal within the unit time is greater than or equal to the quantity of sending times of the second communication signal within the unit time.

In a possible implementation, when performing the determining the first concentration of the first gas based on the transmit power of the first communication signal and the receive power of the first echo signal, the processing unit is configured to:
determine the first concentration of the first gas based on the transmit power of the first communication signal, the receive power of the first echo signal, a distance between the first communication device and the second communication device, and the absorption coefficient of the first gas at the frequency of $f_1$.

In a possible implementation, the first concentration satisfies:

$$M_A = \frac{1}{2L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

In a possible implementation, when performing the determining the second concentration of the first gas based on the transmit power of the first communication signal, the receive power of the first echo signal, the transmit power of the second communication signal, and the receive power of the second echo signal, the processing unit is configured to:
determine the second concentration of the first gas on a communication link between the second communication device and the second communication device based on the transmit power of the first communication signal, the receive power of the first echo signal, the transmit power of the second communication signal, the receive power of the second echo signal, a distance between the first communication device and the second communication device, the absorption coefficient of the first gas at the frequency of $f_1$, and the absorption coefficient of the first gas at the frequency of $f_2$.

In a possible implementation, the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first echo signal, $P_{B0}(f_1)$ represents the transmit power of the second communication signal, and $P_A(f_1)$ represents the receive power of the second echo signal.

In a possible implementation, both $f_1$ and $f_2$ belong to a licensed frequency band, or $f_1$ belongs to a licensed frequency band, and $f_2$ belongs to an unlicensed frequency band.

According to a fifth aspect, this application provides an apparatus for determining a gas concentration. The apparatus is a first communication device, and the apparatus includes:

a transceiver unit, configured to send a first communication signal to a second communication device, where a frequency of the first communication signal is $f_1$, $f_1$ is used for a first gas, and transmit power and receive power of the first communication signal are used to determine a first concentration of the first gas; and

a processing unit, configured to determine, based on first indication information from the second communication device, whether to send a second communication signal to the second communication device, where a frequency of the second communication signal is $f_2$, $f_2$ is used for the first gas, and an absorption coefficient of the first gas at the frequency of $f_1$ is greater than an absorption coefficient of the first gas at the frequency of $f_2$.

In a possible implementation, the first indication information indicates the first communication device to send the second communication signal to the second communication device.

In a possible implementation, the first indication information includes the first concentration.

In a possible implementation, the processing unit is further configured to:
send the first communication signal and the second communication signal to the second communication device.

In a possible implementation, a quantity of sending times of the first communication signal within unit time is greater than or equal to a quantity of sending times of the second communication signal within the unit time.

In a possible implementation, the processing unit is further configured to:
determine, based on second indication information from the second communication device, to stop sending the second communication signal to the second communication device or reduce the quantity of sending times of sending the second communication signal to the second communication device within the unit time.

In a possible implementation, the second indication information indicates to stop sending the second communication signal to the second communication device or reduce the quantity of sending times of sending the second communication signal to the second communication device within the unit time.

In a possible implementation, the second indication information includes the first concentration and/or a second concentration of the first gas, and the second concentration is determined based on the transmit power and the receive power of the first communication signal and transmit power and receive power of the second communication signal.

In a possible implementation, both $f_1$ and $f_2$ belong to a licensed frequency band, or $f_1$ belongs to a licensed frequency band, and $f_2$ belongs to an unlicensed frequency band.

According to a sixth aspect, this application provides an apparatus for determining a gas concentration. The apparatus is a second communication device, and the apparatus includes:

a transceiver unit, configured to receive a first communication signal from a first communication device, where a frequency of the first communication signal is $f_1$, and $f_1$ is used for a first gas; and

a processing unit, configured to determine a first concentration of the first gas based on receive power and transmit power of the first communication signal, where

the processing unit is configured to determine, based on the first concentration, whether to send first indication information to the first communication device, where the first indication information is used to determine that the first communication device sends a second communication signal to the second communication device; or

the transceiver unit is configured to send first indication information to the first communication device, where the first indication information is used to determine whether the first communication device sends a second communication signal to the second communication device, and the first indication information includes the first concentration.

In a possible implementation, when performing the determining, based on the first concentration, whether to send the first indication information to the first communication device, the processing unit is configured to:
determine, based on one of the following, to send the first indication information to the first communication device:

when the first concentration is greater than or equal to a first concentration threshold, the first indication information is determined to be sent to the first communication device; or

when the first concentration is less than a first concentration threshold, and the first indication information is not sent to the first communication device within preset duration, the first indication information is determined to be sent to the first communication device.

In a possible implementation, when performing the determining, based on the first concentration, whether to send the first indication information to the first communication device, the processing unit is configured to:
determine, based on the following condition, not to send the first indication information to the first communication device:
when the first concentration is less than a first concentration threshold, the first indication information is determined not to be sent to the first communication device.

In a possible implementation, the first indication information is used to determine that the first communication device sends the second communication signal to the second communication device;

the transceiver unit is further configured to receive the first communication signal and the second communication signal from the first communication device; and

the processing unit is further configured to determine a second concentration of the first gas based on the transmit power and the receive power of the first communication signal and transmit power and receive power of the second communication signal.

In a possible implementation, the processing unit is further configured to:

when the first concentration is less than the first concentration threshold, send second indication information to the first communication device, where the second indication information indicates the first communication device to stop sending the second communication signal to the second communication device or reduce a quantity of sending times of sending the second communication signal to the second communication device within unit time; or

when the second concentration is less than the first concentration threshold, send second indication information to the first communication device, where the second indication information indicates the first communication device to stop sending the second communication signal to the second communication device or reduce a quantity of sending times of sending the second communication signal to the second communication device within unit time.

In a possible implementation, the processing unit is further configured to:
when a concentration difference between the first concentration and the second concentration is less than or equal to a second concentration threshold, send second indication information to the first communication device, where the second indication information indicates the first communication device to stop sending the second communication signal to the second communication device or reduce a quantity of sending times of sending the second communication signal to the second communication device within unit time.

In a possible implementation, the processing unit is further configured to:
send second indication information to the first communication device, where the second indication information includes the first concentration and/or the second concentration.

In a possible implementation, when performing the determining the first concentration of the first gas based on the receive power and the transmit power of the first communication signal, the processing unit is configured to:
determine the first concentration of the first gas based on the transmit power of the first communication signal, the receive power of the first communication signal, a distance between the second communication device and the first communication device, and an absorption coefficient of the first gas at the frequency of $f_1$.

In a possible implementation, the first concentration satisfies:

$$M_A = \frac{1}{L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the second communication device and the first communication device, $P_{A0}(f_1)$ represents the transmit power of the first communication signal, and $P_A(f_1)$ represents the receive power of the first communication signal.

In a possible implementation, when performing the determining the second concentration of the first gas based on the transmit power and the receive power of the first communication signal and the transmit power and the receive power of the second communication signal, the processing unit is configured to:

determine the second concentration of the first gas on a communication link between the first communication device and the first communication device based on the transmit power of the first communication signal, the receive power of the first communication signal, the transmit power of the second communication signal, the receive power of the second communication signal, a distance between the second communication device and the first communication device, an absorption coefficient of the first gas at the frequency of $f_1$, and an absorption coefficient of the first gas at a frequency of $f_2$.

In a possible implementation, the second concentration satisfies:

$$M_{AB} = \frac{1}{\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the second communication device and the first communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

In a possible implementation, both $f_1$ and $f_2$ belong to a licensed frequency band, or $f_1$ belongs to a licensed frequency band, and $f_2$ belongs to an unlicensed frequency band.

According to a seventh aspect, this application provides a communication apparatus. The apparatus may be a first communication device, or may be an apparatus in a first communication device, or may be an apparatus that can be used together with a first communication device. For example, the first communication device may be a terminal device or an access network device. The communication apparatus may alternatively be a chip system. The communication apparatus may perform the method according to the first aspect or the second aspect. A function of the communication apparatus may be implemented by hardware, or may be implemented by hardware by executing corresponding software. The hardware or software includes one or more units or modules corresponding to the foregoing function. The unit or the module may be software and/or hardware. For operations performed by the communication apparatus and beneficial effect thereof, refer to the method according to the first aspect or the second aspect, and the beneficial effect thereof. Repeated content is not described.

According to an eighth aspect, this application provides a communication apparatus. The apparatus may be a second communication device, or may be an apparatus in a second communication device, or may be an apparatus that can be used together with a second communication device. For example, the second communication device may be a terminal device or an access network device. The communication apparatus may alternatively be a chip system. The communication apparatus may perform the method according to the third aspect. A function of the communication apparatus may be implemented by hardware, or may be implemented by hardware by executing corresponding software. The hardware or software includes one or more units or modules corresponding to the foregoing function. The unit or the module may be software and/or hardware. For operations performed by the communication apparatus and beneficial effect thereof, refer to the method according to the third aspect, and the beneficial effect thereof. Repeated content is not described.

According to a ninth aspect, this application provides a communication apparatus. The apparatus may be a first communication device, the communication apparatus includes a processor and a transceiver, and the processor and the transceiver are configured to execute a computer program or instructions stored in at least one memory, so that the apparatus implements the method according to any one of the implementations of the first aspect or the second aspect.

For another example, the communication apparatus includes a processor, a transceiver, and a memory. The processor, the transceiver, and the memory are coupled. The processor and the transceiver are configured to implement the method according to any one of the implementations of the first aspect or the second aspect.

According to a tenth aspect, this application provides a communication apparatus. The apparatus may be a second communication device, the communication apparatus includes a processor and a transceiver, and the processor and the transceiver are configured to execute a computer program or instructions stored in at least one memory, so that the apparatus implements the method according to any one of the implementations of the third aspect.

For another example, the communication apparatus includes a processor, a transceiver, and a memory. The processor, the transceiver, and the memory are coupled. The processor and the transceiver are configured to implement the method according to any one of the implementations of the third aspect.

According to an eleventh aspect, this application provides a computer-readable storage medium. The storage medium stores a computer program or instructions, and when the computer program or the instructions are executed by a computer, the method according to any one of the implementations of the first aspect to the third aspect is implemented.

According to a twelfth aspect, this application provides a computer program product including instructions. The computer program product includes computer program code, and when the computer program code is run on a computer, the method according to any one of the implementations of the first aspect to the third aspect is implemented.

According to a thirteenth aspect, a chip system is provided. The chip system includes a processor, and may further include a memory, to implement the method according to any one of the first aspect to the third aspect and the possible designs of the first aspect to the third aspect. The chip system may include a chip, or may include a chip and another discrete device.

According to a fourteenth aspect, a communication system is provided. The communication system includes the first communication device according to the seventh aspect or the ninth aspect and the second communication device according to the eighth aspect or the tenth aspect.

## BRIEF DESCRIPTION OF DRAWINGS

FIG. 1 is a diagram of a network architecture of a communication system;
FIG. 2 is a schematic flowchart of a method for determining a gas concentration according to an embodiment of this application;
FIG. 3 is another schematic flowchart of a method for determining a gas concentration according to an embodiment of this application;
FIG. 4 is a diagram of a structure of a communication apparatus according to an embodiment of this application;
FIG. 5 is a diagram of a structure of another communication apparatus according to an embodiment of this application;
FIG. 6 is a diagram of a structure of another communication apparatus according to an embodiment of this application; and
FIG. 7 is a diagram of a structure of another communication apparatus according to an embodiment of this application.

## DESCRIPTION OF EMBODIMENTS

The following clearly and completely describes technical solutions in embodiments of this application with reference to accompanying drawings in embodiments of this application.

In descriptions of this application, unless otherwise specified, "/" means "or". For example, A/B may indicate A or B. A term "and/or" in this specification describes only an association relationship between associated objects and indicates that there may be three relationships. For example, A and/or B may represent the following three cases: Only A exists, both A and B exist, and only B exists. A and B may be single or multiple. In addition, "at least one" means one or more, and "a plurality of" means two or more. "One or more of the following: ...", "at least one of..." and similar expressions thereof mean any combination of the listed items. For example, "one or more of the following: A, B, C" may indicate: There are six cases: only A, only B, only C, both A and B, both B and C, both A and C, and both A, B, and C, where A, B, and C each may be singular or plural. Terms such as "first" and "second" do not limit a quantity and an execution sequence, and the terms such as "first" and "second" do not indicate a definite difference.

In this application, the term "example", "for example", or the like indicates an example, an illustration, or a description. Any embodiment or design scheme described as an "example" or "for example" in this application should not be explained as being more preferred or having more advantages than another embodiment or design scheme. To be precise, use of the word such as "example" or "for example" is intended to present a relative concept in a specific manner.

It should be understood that, an "embodiment" mentioned throughout this specification means that particular features, structures, or characteristics related to this embodiment are included in at least one embodiment of this application. Therefore, embodiments in the entire specification are necessarily a same embodiment. In addition, these particular features, structures, or characteristics may be combined in one or more embodiments in any appropriate manner.

The technical solutions in embodiments of this application may be applied to various communication systems, for example, a long term evolution (long term evolution, LTE) system, a wireless fidelity (wireless fidelity, Wi-Fi) system, a worldwide interoperability for microwave access (worldwide interoperability for microwave access, WiMAX) communication system, a 5th generation (5th generation, 5G) system or a new radio (new radio, NR), and a future communication system. This is not limited herein.

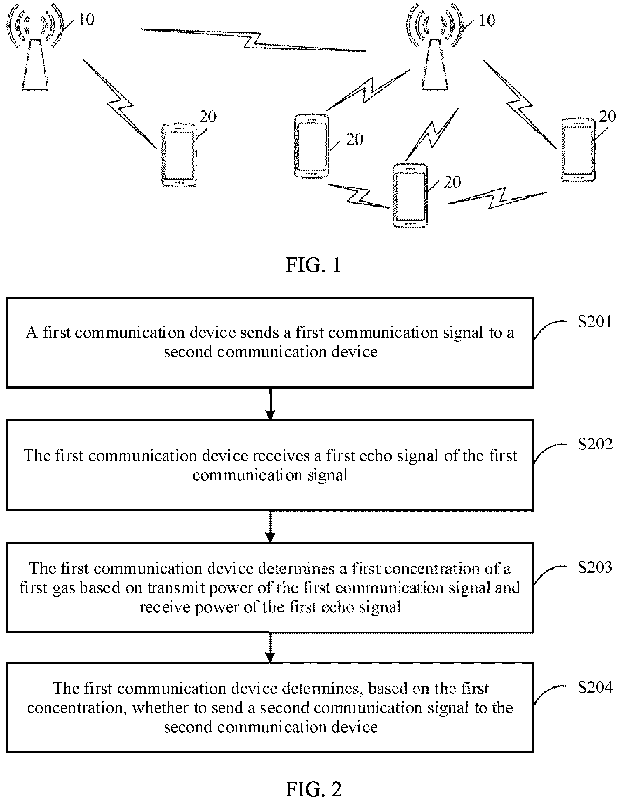

FIG. 1 is a diagram of a network architecture of a communication system. As shown in FIG. 1, the communication system may include one or more network devices 10 (two are shown in FIG. 1) and one or more terminal devices 20 that communicate with each network device 10. FIG. 1 is merely a diagram, and does not constitute any limitation on a scenario to which the technical solutions provided in this application are applicable.

The terminal device includes a device that provides a user with voice and/or data connectivity, for example, may include a handheld device having a wireless connection function, or a processing device connected to a wireless modem. The terminal device may communicate with a core network through a radio access network. The terminal device may include a user device (user equipment, UE), a wireless terminal device, a mobile terminal device, a device-to-device (device-to-device, D2D) terminal device, a vehicle-to-everything (vehicle-to-everything, V2X) terminal device, a machine-to-machine/machine-type communication (machine-to-machine/machine-type communication, M2M/MTC) terminal device, an internet of things (internet of things, IoT) terminal device, a subscriber unit, a subscriber station, a mobile station, a remote station, an access point (access point, AP), a remote terminal, an access terminal, a user terminal, a user agent, user equipment, or the like. For example, the terminal device may include a mobile phone (or referred to as a "cellular" phone), a computer with a mobile terminal device, or a portable, pocket-sized, handheld, or computer built-in mobile apparatus, for example, a personal communication service (personal communication service, PCS) phone, a cordless phone, a session initiation protocol (session initiation protocol, SIP) phone, a wireless local loop (wireless local loop, WLL) station, a personal digital assistant (personal digital assistant, PDA), or another device. The terminal device may further include a limited device, for example, a device with low power consumption, a device with a limited storage capability, or a device with a limited computing capability.

The network device may be a core network device, for example, a core network (core network, CN) device, or may be an access network device, for example, a radio access network (radio access network, RAN) device, or may include both a core network device and an access network device.

Usually, the core network device is configured to implement functions such as mobility management, data processing, session management, and policy and charging. Names of devices that implement functions of the core network in systems of different access technologies may be different. This is not limited in this application. A 5G network is used as an example. A logical network element of a 5GC includes an access and mobility management function (access and mobility management function, AMF), a session management function (session management function, SMF), a user plane function (user plane function, UPF), or the like.

In embodiments of this application, an apparatus configured to implement a function of the core network device may be the core network device, or may be an apparatus that can support the core network device in implementing the function, for example, a chip system, or a combination device or a component that can implement a function of the core network device. The apparatus may be installed in the core network device.

Usually, the access network device may include a next-generation base station (generation NodeB, gNB), an evolved NodeB (evolved NodeB, eNB), a next-generation evolved NodeB (next generation eNB, ng-eNB), a wireless backhaul device, a radio network controller (radio network controller, RNC), a NodeB (NodeB, NB), a base station controller (base station controller, BSC), a base transceiver station (base transceiver station, BTS), a home base station ((home evolved NodeB, HeNB) or (home NodeB, HNB)), a baseband unit (baseband unit, BBU), a transmission reception point (transmitting and receiving point, TRP), a transmission point (transmitting point, TP), a mobile switching center, and the like in a 5G communication system. This is not limited herein.

The access network device may further include one or more central units (central units, CUs), one or more distributed units (distributed units, DUs), or one or more CUs and one or more DUs. For example, a function of the CU may be implemented by one entity or different entities. For example, the function of the CU is further divided. In other words, a control plane and a user plane are separated and implemented by using different entities: a control plane CU entity (namely, a CU-CP entity) and a user plane CU entity (namely, a CU-UP entity). The CU-CP entity and the CU-UP entity may be coupled to the DU, to jointly complete a function of an access network device. In this way, some functions of a radio access network device may be implemented by a plurality of network function entities. These network function entities may be network elements in a hardware device, or may be software functions run on dedicated hardware, or may be virtualized functions instantiated on a platform (for example, a cloud platform).

In embodiments of this application, an apparatus configured to implement the function of the access network device may be the access network device, or may be an apparatus that can support the access network device in implementing the function, for example, a chip system, or a combination device or component that can implement the function of the access network device. The apparatus may be installed in the access network device. In embodiments of this application, the chip system may include a chip, or may include a chip and another discrete device.

It may be understood that a scenario in embodiments of this application may be a background based on communication signal transmission. Optionally, embodiments of this application are applicable to both a homogeneous network scenario and a heterogeneous network scenario, and a transmission point is not limited in embodiments of this application. The transmission point may be coordinated multipoint transmission between a macro base station and a macro base station, between a micro base station and a micro base station, or between a macro base station and a micro base station, and is applicable to a frequency division duplex FDD (frequency division duplex)/TDD system. Optionally, embodiments of this application are applicable to a low-frequency scenario (sub 6G), and are also applicable to a high-frequency scenario (above 6G). Optionally, embodiments of this application are applicable to a 4G, 5G, or future mobile communication system. Optionally, embodiments of this application are applicable to a single-TRP scenario or a multi-TRP scenario, and any scenario derived from the single-TRP scenario or the multi-TRP scenario. Optionally, this application is also applicable to NR uplink transmission and the like.

It should be noted that a first communication device described in embodiments of this application may be a network device, or may be a terminal device, and a second communication device may be a network device, or may be a terminal device, or the like. This is specifically determined based on an actual application scenario, and is not limited herein. For example, a network architecture to which this application is applicable may include the following six types:

① A network device 1 sends a communication signal, and the network device 1 receives an echo signal of the communication signal sent by the network device 1.
② A terminal device 1 sends a communication signal, and the terminal device 1 receives an echo signal of the communication signal sent by the terminal device 1.
③ The network device 1 sends a communication signal, and a network device 2 receives the communication signal.
④ The terminal device 1 sends a communication signal, and a terminal device 2 receives the communication signal.
⑤ The network device 1 sends a communication signal, and the terminal device 1 receives the communication signal.
⑥ The terminal device 1 sends a communication signal, and the network device 1 receives the communication signal.

Customers have requirements for a gas detection function in multiple application scenarios. For example, in industrial scenarios, toxic gases, corrosive gases, and explosive gases are focused on, and a production line progress can be effectively monitored by detecting a gas that is used as a primary (secondary) product; in environment monitoring, air quality can be monitored in real time by detecting a concentration of a specific gas in an atmosphere, which facilitates an environmental supervision department to targetedly carry out an air pollution prevention and control solution; in agriculture and animal husbandry, gases released by crops and livestock represents life cycles or physiological statuses of the crops and livestock, and detecting these gases helps practitioners detect the growth of the crops and livestock in time, to adjust care measures in an early manner and improve a production value; in combination with the national strategy of "dual carbon", a gas detection function of the communication system may be used to detect a large quantity of greenhouse gases released by the transportation, agriculture, and energy industries, to provide data support for calculating indicators of carbon neutrality; in daily life, toxic gases such as carbon monoxide poisoning events that occur frequently in winter are detected in real time, to reduce losses caused by safety accidents in daily life; and real-time monitoring of explosive gas leakage helps avoid fire accidents in time.

However, currently, gas detection requires a dedicated gas detection device. Due to economic costs, a distribution density of an instrument with gas detection function is low, and is insufficient to provide a data source with a sufficient spatial-temporal distribution density. In addition, in special scenarios (such as in home or in a dangerous workplace), the market is more sensitive to features such as simplicity, portability (wearability), and cost-effectiveness of a gas detection apparatus. However, a current dedicated gas detection device cannot meet customer requirements in terms of cost-effectiveness and operability. Based on this, an integrated sensing and communication (Integrated sensing and communication, ISAC) technology is proposed in a 6G vision, in other words, a future communication system integrates two functions, namely, communication and sensing functions at the same time, and the two functions promote and enhance each other through an integrated design of waveforms and components. Potential application scenarios of the ISAC sensing function proposed in the 6G vision include air quality detection, personal health monitoring, gesture recognition, and security check and disaster prevention. Gas detection is one of potential functions of 6G base stations or terminal devices. Therefore, it is necessary to make a basic general design based on function features and application scenarios of gas detection in a wireless communication system.

It should be noted that, in embodiments of this application, a concentration estimated based on only a first communication signal is described as a first concentration, a concentration estimated based on the first communication signal and a second communication signal is described as a second concentration, and a concentration estimated based on only the second communication signal is described as a third concentration.

It should be noted that, in embodiments of this application, sending the first communication signal may be understood as enabling a module A of the first communication device to send the first communication signal, and sending the second communication signal may be understood as enabling a module B of the first communication device to send the second communication signal.

It may be understood that, when the module A and the module B are simultaneously enabled, a quantity of sending times of sending the first communication signal by the module A within unit time may be equal to a quantity of sending times of sending the second communication signal by the module B within the unit time, in other words, a transmit frequency of sending the first communication signal by the module A is equal to a transmit frequency of sending the second communication signal by the module B, that is, the module A and the module B send the signals synchronously. Herein, the unit time may be determined based on an actual scenario. For example, the unit time may be 5 minutes, one quarter, or one hour. This is not limited herein.

Optionally, when the module A and the module B are both enabled, a quantity of sending times of sending the first communication signal by the module A within unit time may alternatively be greater than a quantity of sending times of sending the second communication signal by the module B within the unit time, in other words, the module A and the module B do not synchronously send the signals, and a transmit frequency of sending the first communication signal by the module A is greater than a transmit frequency of sending the second communication signal by the module B. This is specifically determined based on an actual scenario, and is not limited herein.

It should be noted that, in embodiments of this application, when a concentration of the first gas is estimated, a module (for example, the module A) configured to send the first communication signal may be in an always enabled state and operate continuously. A module (for example, the module B) configured to send the second communication signal may determine, based on an actual situation, whether to be enabled and whether the module operates continuously or intermittently operates after being enabled. Herein, the module A and the module B operating continuously may be understood as that the module A and the module B send the signals synchronously. The module A operating continuously but the module B operating intermittently may be understood as that the module A and the module B do not send the signals synchronously, and the transmit frequency of sending the first communication signal by the module A is greater than the transmit frequency of sending the second communication signal by the module B.

It should be noted that, in embodiments of this application, $f_1$ and $f_2$ both belong to a licensed frequency band, or $f_1$ belongs to a licensed frequency band, and $f_2$ belongs to an unlicensed frequency band. Herein, the licensed frequency band is a frequency band that has been allocated to an operator to which a cellular network belongs, and that is used only for communication and that is not occupied for another purpose. The unlicensed frequency band is a frequency band that is not allocated to an operator to which a cellular network belongs. Generally, for the unlicensed frequency band, there are two cases. A first case is that the unlicensed frequency band is occupied for another purpose and is not allowed to be used for a communication purpose, and a second case is that the unlicensed frequency band is a frequency band that can be used for communication and shared with another purpose. In this application, if $f_2$ belongs to the unlicensed frequency band, $f_2$ belongs to the second case.

It should be noted that a gas concentration (for example, the first concentration) described in embodiments of this application may be understood as a gas mass concentration or a gas volume concentration. The gas mass concentration is a mass quantity of a specific gas molecule in a unit volume. A unit is mg per cubic meter ($mg/m^3$) or $g/m^3$. The gas volume concentration is a volume quantity of a specific gas molecule in a unit volume, or a volume percentage of a measured gas in a specific volume of a gas sample. A unit is %, parts per million (ppm), ppb (parts per billion), ppt (parts per trillion), or the like. For example, 2 ppm methane means that two of every million gas molecules are methane. Optionally, the gas concentration (for example, the first concentration) may also be understood as an average value of the gas mass concentration or the gas volume concentration within unit time. The unit time herein may be 5 minutes, one minute, one hour, or the like. This may be specifically determined based on an application scenario, and is not limited herein. It may be understood that, in this application, the gas concentration is calculated continuously, in other words, a gas concentration value is updated continuously, for example, updated once per unit time. This is not limited herein.

The following describes in detail a method for determining a gas concentration and a communication apparatus that are provided in this application.

FIG. 2 is a schematic flowchart of a method for determining a gas concentration according to an embodiment of this application. It should be noted that the method for determining a gas concentration provided in FIG. 2 is applicable to the foregoing system architecture ① and system architecture ②. As shown in FIG. 2, the method for determining a gas concentration includes the following steps S201 to S204.

S201: A first communication device sends a first communication signal to a second communication device.

Herein, a frequency of the first communication signal is $f_1$, and $f_1$ is used for/corresponds to a first gas. It may be understood that a category of a gas that can be detected by a wireless communication system depends on a frequency band allocated by a local operator. Generally, different gases have different absorption degrees for signals in different frequency bands/frequencies. Generally, an absorption degree of a gas for a signal at a frequency may be measured by an absorption coefficient. Generally, for a gas, a larger absorption coefficient at a frequency indicates stronger absorption of the gas at the frequency. The absorption coefficient may be measured through an experiment.

In an implementation, the first communication device sending the first communication signal to the second communication device may be understood as that the first communication device sends the first communication signal to the second communication device through a module A.

S202: The first communication device receives a first echo signal of the first communication signal.

Generally, the first communication signal sent by the first communication device may be absorbed by a gas (for example, the first gas) in a surrounding environment, and reflected back to the first communication device by another device or an object in the surrounding environment. In other words, the first communication device may receive a reflected signal of the first communication signal. For ease of description, the reflected signal is described as the first echo signal in this application.

In an implementation, the first communication device receiving the first echo signal of the first communication signal may be understood as that the first communication device receives the first echo signal through the module A.

S203: The first communication device determines a first concentration of the first gas based on transmit power of the first communication signal and receive power of the first echo signal.

In some feasible implementations, the first communication device may determine the first concentration of the first gas based on the transmit power of the first communication signal, the receive power of the first echo signal, a distance between the first communication device and the second communication device, and an absorption coefficient of the first gas at the frequency of $f_1$. For example, the first concentration satisfies:

$$M_A = \frac{1}{2L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

S204: The first communication device determines, based on the first concentration, whether to send a second communication signal to the second communication device.

In some feasible implementations, the first communication device may determine, based on the first concentration, whether to send the second communication signal to the second communication device. When the first communication device determines, based on the first concentration, to send the second communication signal to the second communication device, the first communication device may specifically send the second communication signal to the second communication device through a module B.

The first communication device may determine, based on one of the following, to send the second communication signal to the second communication device.

1. When the first concentration is greater than or equal to a first concentration threshold, the second communication signal is determined to be sent to the second communication device; or

2. When the first concentration is less than a first concentration threshold and the second communication signal is not sent to the second communication device within preset duration, the second communication signal is determined to be sent to the second communication device. In other words, if the module B is always in a disabled state, and the module B does not receive an enabling instruction within specific duration after a time point at which the module B is disabled last time, the module B is automatically enabled, and receives or sends a signal synchronously with the module A.

Optionally, the first communication device may determine, based on one of the following, not to send the second communication signal to the second communication device: When the first concentration is less than a first concentration threshold, the first communication device determines not to send the second communication signal to the second communication device.

It should be noted that, when the second communication signal is determined, based on the first concentration, to be sent the second communication device, the first communication device may send the first communication signal and the second communication signal to the second communication device, and receive the first echo signal of the first communication signal and a second echo signal of the second communication signal. Herein, the first communication device sending the first communication signal and the second communication signal to the second communication device may be understood as that the first communication device sends the first communication signal to the second communication device through the module A, and the first communication device sends the second communication signal to the second communication device through the module B. A quantity of sending times of sending the first communication signal by the module A within unit time is equal to a quantity of sending times of sending the second communication signal by the module B within the unit time. Correspondingly, the first communication device receiving the first echo signal of the first communication signal and the second echo signal of the second communication signal may be understood as that the first communication device receives the first echo signal through the module A, and receives the second echo signal through the module B.

A frequency of the second communication signal is $f_2$, $f_2$ is used for the first gas, and the absorption coefficient of the first gas at the frequency of $f_1$ is greater than an absorption coefficient of the first gas at the frequency of $f_2$, in other words, an attenuation degree of the first communication signal whose frequency is $f_1$ is more obvious than an attenuation degree of the second communication signal whose frequency is $f_2$, and sensitivity is higher. Further, the first communication device may determine a second concentration of the first gas based on the transmit power of the first communication signal, the receive power of the first echo signal, transmit power of the second communication signal, and receive power of the second echo signal. For example, that the second concentration of the first gas is determined based on the transmit power of the first communication signal, the receive power of the first echo signal, the transmit power of the second communication signal, and the receive power of the second echo signal may be understood as that the first communication device determines the second concentration of the first gas on a communication link between the first communication device and the second communication device based on the transmit power of the first communication signal, the receive power of the first echo signal, the transmit power of the second communication signal, the receive power of the second echo signal, the distance between the first communication device and the second communication device, the absorption coefficient of the first gas at the frequency of $f_1$, and the absorption coefficient of the first gas at the frequency of $f_2$. For example, the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first echo signal, $P_{B0}(f_1)$ represents the transmit power of the second communication signal, and $P_A(f_1)$ represents the receive power of the second echo signal.

Optionally, when the first communication device sends the second communication signal, the first communication device may further determine a third concentration of the first gas based on the transmit power of the second communication signal and the receive power of the second echo signal. For example, the third concentration of the first gas may be determined based on the transmit power of the second communication signal, the receive power of the second echo signal, the distance between the first communication device and the second communication device, and the absorption coefficient of the first gas at the frequency of $f_2$. For example, the third concentration satisfies:

$$M_B = \frac{1}{2L \cdot \sigma(f_2)} \ln \frac{P_{B0}(f_2)}{P_B(f_2)},$$

where $M_B$ represents the third concentration, $\sigma(f_2)$ represents the absorption coefficient of the first gas at the frequency of $f_2$, $L$ represents the distance between the first communication device and the second communication device, $P_{B0}(f_2)$ represents the transmit power of the second communication signal, and $P_B(f_2)$ represents the receive power of the second echo signal.

In general, in a same gas environment, a concentration (for example, the second concentration $M_{AB}$) calculated based on communication signals of two different frequencies (for example, $f_1$ and $f_2$) within same unit time is more accurate than a concentration (for example, the first concentration $M_A$ or the third concentration $M_B$) calculated based on communication signals of only one frequency (for example, $f_1$ or $f_2$). In addition, because both the frequency $f_1$ and the frequency $f_2$ are within a communication frequency band corresponding to the first gas, a criterion for selecting $f_1$ and $f_2$ is to satisfy: $\sigma(f_1) > \sigma(f_2)$, that is, a received or sent signal whose frequency is $f_1$ has a more obvious attenuation degree and higher sensitivity. Therefore, $M_A$ is generally more accurate than $M_a$ by default. Therefore, when the module A and the module B are enabled at the same time, even if $M_{AB}$, $M_y$, and $M_B$ can be calculated separately, it is more likely that the output $M_{AB}$ is used to represent an average gas concentration on the link, $M_A$ is second choice, and $M_B$ is the last choice. However, $M_{AB}$ requires two modules with different frequencies to operate synchronously, and consumes more resources. Therefore, when a precision requirement for a gas concentration is not high, a received or sent signal (for example, the first communication signal and the first echo signal) of one module (for example, the module A) may be used to calculate the gas concentration $M_A$; or when a precision requirement for a gas concentration is high, received or sent signals (for example, the first communication signal, the first echo signal, the second communication signal, and the second echo signal) of two modules (for example, the module A and the module B) need to be used to calculate the gas concentration $M_{AB}$ according to a differential absorption algorithm.

In this embodiment of this application, a condition in which the gas concentration is calculated based on a received or sent signal of only one module may be referred to as a general operating condition, and a condition in which the gas concentration is calculated based on synchronously received or sent signals of two modules is referred to as a warning operating condition. For example, for flammable and explosive gases, users are more concerned that the concentrations of these gases exceed a specific upper limit, which poses a threat to production, life, and personnel health and safety. Therefore, the gas concentration is far lower than a specific warning value, which has a low requirement for measurement precision of a gas concentration. In this way, it is unnecessary to use excessive resources to monitor the gas concentration. Therefore, generally, when the gas concentration is low, fewer resources are used to monitor the gas concentration, and the system is in the general operating condition. When the gas concentration is high, more resources are used to improve the precision of monitoring the gas concentration, and the system is in the warning operating condition. Optionally, in some special scenarios, there is an exact opposite requirement, that is, more resources need to be scheduled to measure the gas concentration only when the gas concentration is lower than a specific warning value. For a scenario in which more resources need to be scheduled to measure the gas concentration only when the gas concentration is lower than a specific warning value, only the determining condition in the embodiment needs to be changed to an opposite meaning. For ease of understanding, in embodiments of this application, an example in which more resources need to be scheduled to measure the gas concentration only when the gas concentration is higher than a specific warning value is mainly used for description.

Optionally, in some feasible implementations, after the first communication device sends the first communication signal and the second communication signal to the second communication device, the first communication device may further determine, based on the first concentration and/or the second concentration estimated in this period of time, whether to stop or slow down sending the second communication signal. For example, the first communication device may determine, based on one of the following, to stop or slow down sending the second communication signal:

1. When the first concentration is less than the first concentration threshold, stopping sending the second communication signal to the second communication device or reducing the quantity of sending times of sending the second communication signal to the second communication device within unit time;
2. When the second concentration is less than the first concentration threshold, stopping sending the second communication signal to the second communication device or reducing the quantity of sending times of sending the second communication signal to the second communication device within unit time; or
3. When a concentration difference between the first concentration and the second concentration is less than or equal to a second concentration threshold, stopping sending the second communication signal to the second communication device or reducing the quantity of sending times of sending the second communication signal to the second communication device within unit time. In other words, when the module A and the module B are enabled at the same time, the first concentration $M_{\{A\}}$ is calculated based on a signal separately received or sent by the module A, and the second concentration $M_{\{A, B\}}$ is calculated based on signals jointly received or sent by the module A and the module B, and $M_{\{A\}}$ and $M_{\{A, B\}}$ are compared. If a difference between the two is reduced to a specific concentration threshold (that is, the second concentration threshold) and can be maintained for a specific period of time, the module B is automatically disabled or slows down the transmit frequency of the second communication signal. Optionally, $\varepsilon =$

$$\frac{|M_{\{A, B\}} - M_{\{A\}}|}{M_{\{A, B\}}} \times 100\%$$

may also be defined. When $\varepsilon$ is less than or equal to a preset percentage, the second communication signal to the second communication device is stopped sending or the quantity of sending times of sending the second communication signal to the second communication device within the unit time is reduced.

Optionally, the first communication device may also determine, based on one of the following, not to stop sending the second communication signal to the second communication device or not to reduce the quantity of sending times of sending the second communication signal to the second communication device within unit time.

1. When the first concentration is greater than or equal to a first concentration threshold, the first communication device determines not to stop sending the second communication signal to the second communication device or not to reduce the quantity of sending times of sending the second communication signal to the second communication device within the unit time;
2. When the second concentration is greater than or equal to a first concentration threshold, the first communication device determines not to stop sending the second communication signal to the second communication device or not to reduce the quantity of sending times of sending the second communication signal to the second communication device within the unit time; or
3. When a concentration difference between the first concentration and the second concentration is greater than a second concentration threshold, the first communication device determines not to stop sending the second communication signal to the second communication device or not to reduce the quantity of sending times of sending the second communication signal to the second communication device within the unit time.

It should be noted that, the stopping sending the second communication signal to the second communication device may be understood as disabling the module B, that is, not sending the second communication signal, or that the quantity of sending times of sending the second communication signal to the second communication device within the unit time is 0.

It should be noted that, the reducing the quantity of sending times of sending the second communication signal to the second communication device within the unit time may be understood as not completely disabling the module B, but different from continuous operating of the module A. The module B operates intermittently, that is, within the same unit time, the quantity of times of sending the second communication signal by the module B at the frequency $f_2$ is apparently less than the quantity of times of sending the first communication signal by the module A at the frequency $f_1$. For example, the module A receives or sends a signal whose carrier frequency is $f_1$ at a frequency of once every minute to calculate the gas concentration, and the module B receives or sends a signal whose carrier frequency is $f_2$ at a frequency of once every 10 minutes to calculate the gas concentration.

It should be noted that, the not stopping sending the second communication signal to the second communication device or not reducing the quantity of sending times of sending the second communication signal to the second communication device within the unit time may be understood as that the module B continuously sends the second communication signal to the second communication device, that is, the module A and the module B synchronously send the corresponding communication signal.

It may be understood that, in a general operating condition, when only the module A receives or sends signals but the module B does not receive or send signals, or when the module A receives or sends signals but the module B slows down receiving or sending a signal compared with the module A, only the signals received or sent by the module A may be used for calculating $M_{\{A\}}$ separately. Optionally, the signal data last updated by the module B at a previous moment and the signal that is received or sent by the module A and that is updated in real time may be used for calculating $M'_{\{A, B\}}$ together. For example, the module A receives or sends signals whose carrier frequencies are $f_1$ at moments 08:10, 08:11, 08:12, and 08:13, and the module B receives or sends a signal whose carrier frequency is $f_2$ only at the moment 08:10. At the moments 08:11, 08:12, and 08:13, because the module B does not receive or send the signal whose carrier frequency is $f_2$, the signal received or sent by the module B at the moment 08:10 may be used for calculating $M'_{\{A, B\}}$. Generally, when $M_{\{A\}}$ or $M'_{\{A, B\}}$ is greater than the first concentration threshold, a warning operating condition needs to be entered. Therefore, the module B may be controlled to increase the quantity of times of receiving or sending signals, so that the module B and the module A synchronously receive or send signals. In this way, subsequently, the signal data synchronously received or sent by the module A and the module B may be used for calculating $M_{\{A, B\}}$ together. Optionally, in the warning operating condition, only the signals received or sent by the module A may be used for calculating $M_{\{A\}}$ separately. For example, at the moment when the module A and the module B receive or send signals synchronously, the two signals whose the carrier frequencies are $f_1$ and $f_2$ are received or sent by the module A and the module B may be used for calculating $M_{\{A, B\}}$, and the signals received or sent by the module A may be used for calculating $M_{\{A\}}$ separately. Therefore, when the calculated $M_{\{A, B\}}$ or $M_{\{A\}}$ is less than the first concentration threshold or the concentration difference between $M_{\{A, B\}}$ and $M_{\{A\}}$ is less than or equal to the second concentration threshold in the warning operating condition, the module B may be controlled to stop operating or restore to an intermittent operating state, and this cycle begins. In other words, when the gas concentration is low, the module B participates in gas concentration calculation with reduced workloads and resources; when the gas concentration is high, the module B and the module A have the same participation, and the entire system participates in gas concentration estimation with a large workload. This improves precision and accuracy of gas measurement.

It may be understood that enabling or disabling of the module B may be enabling the module B by using an enabling instruction sent by the module A, or the disabling of the module B may be disabling the module B by using a disabling instruction sent by the module A. Optionally, enabling or disabling of the module A and/or the module B may also be controlled by a module independent of the module A and the module B, for example, a module configured to calculate a concentration. This is not limited herein. It should be noted that when the modules are located inside the first network device, interaction between the modules may be understood as an implementation inside a network device.

In this embodiment of this application, the first communication device is not only a transmit end of a communication signal, but also a receive end of an echo signal of the communication signal. The first communication device calculates a gas concentration based on transmit power of a sent communication signal and receive power of a received echo signal, so that a gas concentration can be estimated, thereby reducing costs of estimating the gas concentration. In addition, the first communication device may further determine, based on the first concentration calculated based on the signal received or sent by the module A, to enable or disable the module B. This not only considers a requirement for calculation precision of a gas concentration, but also helps save resources.

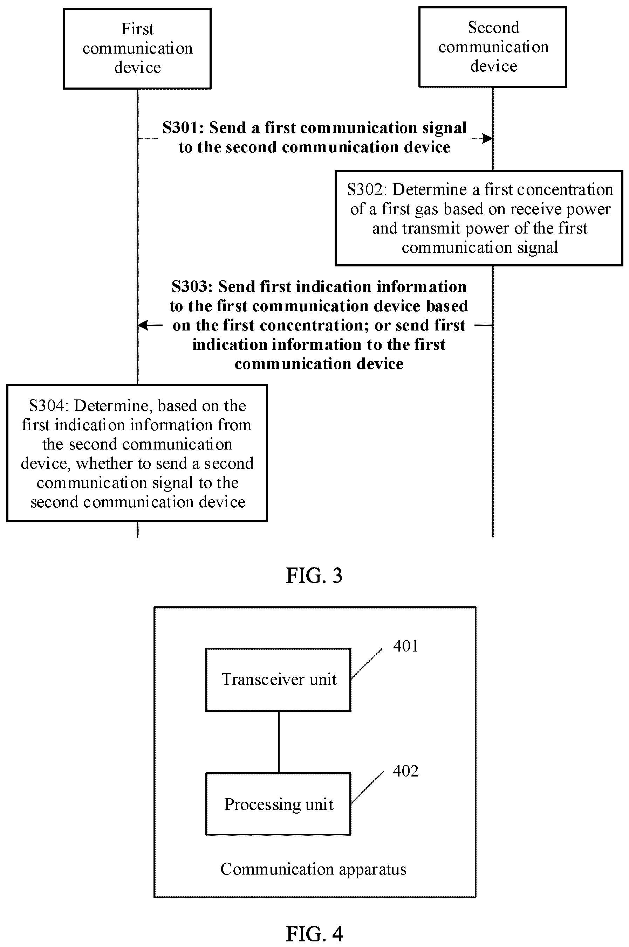

FIG. 3 is another schematic flowchart of a method for determining a gas concentration according to an embodiment of this application. It should be noted that the method for determining a gas concentration provided in FIG. 3 is applicable to the foregoing system architecture ③ to system architecture ⑥. As shown in FIG. 3, the method for determining a gas concentration includes the following steps S301 to S304.

S301: A first communication device sends a first communication signal to a second communication device. Correspondingly, the second communication device receives the first communication signal from the first communication device.

Herein, a frequency of the first communication signal is $f_1$, and $f_1$ is used for/corresponds to a first gas. It may be understood that a category of a gas that can be detected by a wireless communication system depends on a frequency band allocated by a local operator. Generally, different gases have different absorption degrees for signals in different frequency bands/frequencies. Generally, an absorption degree of a gas for a signal at a frequency may be measured by an absorption coefficient. Generally, for a gas, a larger absorption coefficient at a frequency indicates stronger absorption of the gas at the frequency. The absorption coefficient may be measured through an experiment.

In an implementation, that the first communication device sends the first communication signal to the second communication device may be understood as that the first communication device sends the first communication signal to the second communication device through a module A.

S302: The second communication device determines a first concentration of the first gas based on receive power and transmit power of the first communication signal.

In some feasible implementations, the second communication device may determine the first concentration of the first gas based on the transmit power of the first communication signal, the receive power of the first communication signal, a distance between the first communication device and the second communication device, and an absorption coefficient of the first gas at the frequency of $f_1$. For example, the first concentration satisfies:

$$ M_A = \frac{1}{L \cdot \sigma(f_1)} \ln \frac{P_{A0}(f_1)}{P_A(f_1)}, $$

where $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first communication signal.

It should be noted that, the transmit power of the first communication signal may be included in the first communication signal, or the transmit power of the first communication signal may also be indicated to the second communication device through additional signaling. This is not limited herein.

S303: The second communication device sends first indication information to the first communication device based on the first concentration; or the second communication device sends first indication information to the first communication device.

In an example, the second communication device may determine, based on the first concentration, whether to send the first indication information to the first communication device. It should be noted that the second communication device may determine, based on one of the following, to send the first indication information to the first communication device.

1. When the first concentration is greater than or equal to a first concentration threshold, the second communication device determines to send the first indication information to the first communication device, where the first indication information is used to determine/indicates the first communication device to send a second communication signal to the second communication device; or

2. When the first concentration is less than a first concentration threshold, and the first indication information is not sent to the first communication device within preset duration, the second communication device determines to send the first indication information to the first communication device. The first indication information is used to determine/indicates the first communication device to send a second communication signal to the second communication device.

Optionally, the second communication device may further determine, based on the following condition, not to send the first indication information to the first communication device: When the first concentration is less than a first concentration threshold, determine not to send the first indication information to the first communication device.

It should be noted that when the second communication device determines to send the first indication information to the first communication device, the second communication device sends the first indication information to the first communication device; or when the second communication device determines not to send the first indication information to the first communication device, the second communication device does not send the first indication information to the first communication device. In the following embodiments of this application, an example in which the second communication device sends the first indication information to the first communication device is mainly used for description. Correspondingly, the first communication device may receive the first indication information from the second communication device.

In another example, the second communication device may directly send the first indication information to the first communication device, where the first indication information is used to determine whether the first communication device sends the second communication signal to the second communication device, and the first indication information includes the first concentration. In other words, when the first indication information directly includes the first concentration, regardless of a value of the first concentration, the first indication information needs to be sent to the first communication device; or when the first indication information includes the first concentration, the second communication device does not need to determine, based on the first concentration, whether to send the first indication information to the first communication device, but sends the first indication information to the first communication device in real time or periodically, the first indication information is used by the first communication device to determine whether the first communication device sends the second communication signal to the second communication device.

S304: The first communication device determines, based on the first indication information from the second communication device, whether to send the second communication signal to the second communication device.

In an example, when the second communication device determines, based on the first concentration, to send the first indication information to the first communication device, the first indication information indicates the first communication device to send the second communication signal to the second communication device. Therefore, after the first communication device receives the first indication information from the second communication device, the first communication device may determine, based on the first indication information, to send the second communication signal to the second communication device. For example, when the first indication information is 1, the first indication information indicates the first communication device to send the second communication signal to the second communication device. For another example, when the first indication information is 0, the first indication information indicates the first communication device to send the second communication signal to the second communication device.

In another example, the first indication information may directly include the first concentration. Therefore, after the first communication device receives the first concentration, the first communication device needs to determine, based on the received first concentration, whether to send the second communication signal to the second communication device. Generally, when the first indication information includes the first concentration, the first communication device may determine, based on one of the following, to send the second communication signal to the second communication device.

1. When the first concentration is greater than or equal to the first concentration threshold, the first communication device determines to send the second communication signal to the second communication device; or
2. When the first concentration is less than the first concentration threshold, and the second communication signal is not sent to the second communication device within the preset duration, the first communication device determines to send the second communication signal to the second communication device.

Optionally, the first communication device may determine, based on one of the following, not to send the second communication signal to the second communication device: When the first concentration is less than the first concentration threshold, the first communication device determines not to send the second communication signal to the second communication device.

It should be noted that, when the first communication device determines to send the second communication signal to the second communication device, the first communication device sends the first communication signal and the second communication signal to the second communication device. When the first communication device determines not to send the second communication signal to the second communication device, the first communication device does not send the first communication signal and the second communication signal to the second communication device. For ease of understanding, the following embodiments of this application are mainly described by using an example in which the first communication device sends the first communication signal and the second communication signal to the second communication device. It can be understood that, the first communication device may specifically send the first communication signal to the second communication device through the module A, and send the second communication signal to the second communication device through a module B. A quantity of sending times of sending the first communication signal by the module A within unit time is equal to a quantity of sending times of sending the second communication signal by the module B within the unit time. Correspondingly, the second communication device receives the first communication signal and the second communication signal from the first communication device.

A frequency of the second communication signal is $f_2$, $f_2$ is used for the first gas, and the absorption coefficient of the first gas at the frequency of $f_1$ is greater than an absorption coefficient of the first gas at the frequency of $f_2$, in other words, an attenuation degree of the first communication signal whose frequency is $f_1$ is more obvious than an attenuation degree of the second communication signal whose frequency is $f_2$, and sensitivity is higher. Therefore, the second communication device may determine a second concentration of the first gas based on the transmit power and the receive power of the first communication signal, and transmit power and receive power of the second communication signal. It should be noted that, the transmit power of the second communication signal may be included in the second communication signal, or the transmit power of the second communication signal may also be indicated to the second communication device through additional signaling. This is not limited herein.

For example, that the second communication device determines the second concentration of the first gas based on the transmit power of the first communication signal, the receive power of the first communication signal, the transmit power of the second communication signal, and the receive power of the second communication signal may be understood as that the second communication device determines the second concentration of the first gas on a communication link between the first communication device and the second communication device based on the transmit power of the first communication signal, the receive power of the first communication signal, the transmit power of the second communication signal, the receive power of the second communication signal, the distance between the first communication device and the second communication device, the absorption coefficient of the first gas at the frequency of $f_1$, and the absorption coefficient of the first gas at the frequency of $f_2$. For example, the second concentration satisfies:

$$M_{AB} = \frac{1}{\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

Optionally, when the first communication device sends the second communication signal, the second communication device may further determine a third concentration of the first gas based on the transmit power of the second communication signal and the receive power of the second communication signal. For example, the third concentration of the first gas may be determined based on the transmit power of the second communication signal, the receive power of the second communication signal, the distance between the first communication device and the second communication device, and the absorption coefficient of the first gas at the frequency of $f_2$. For example, the third concentration satisfies:

$$M_B = \frac{1}{L \cdot \sigma(f_2)} \ln \frac{P_{B0}(f_2)}{P_B(f_2)},$$

where $M_B$ represents the third concentration, $\sigma(f_2)$ represents the absorption coefficient of the first gas at the frequency of $f_2$, $L$ represents the distance between the first communication device and the second communication device, $P_{B0}(f_2)$ represents the transmit power of the second communication signal, and $P_B(f_2)$ represents the receive power of the second communication signal.

In general, in a same gas environment, a concentration (for example, the second concentration $M_{AB}$) calculated based on communication signals of two different frequencies (for example, $f_1$ and $f_2$) within same unit time is more accurate than a concentration (for example, the first concentration $M_A$ or the third concentration $M_B$) calculated based on communication signals of only one frequency (for example, $f_1$ or $f_2$). In addition, because both the frequency $f_1$ and the frequency $f_2$ are within a communication frequency band corresponding to the first gas, a criterion for selecting $f_1$ and $f_2$ is to satisfy: $\sigma(f_1) > \sigma(f_2)$, that is, a communication signal whose frequency is $f_1$ has a more obvious attenuation degree and higher sensitivity. Therefore, $M_A$ is generally more accurate than $M_B$ by default. Therefore, when the module A and the module B of the first communication device are enabled at the same time, even if $M_{AB}$, $M_A$, and $M_B$ can be calculated separately, it is more likely that the output $M_{AB}$ is used to represent an average gas concentration on the link, $M_A$ is second choice, and $M_B$ is the last choice. However, $M_{AB}$ requires two modules with different frequencies to operate synchronously, and consumes more resources. Therefore, when a precision requirement for the gas concentration is not high, a communication signal (for example, the first communication signal) of one module (for example, the module A) may be used to calculate the gas concentration $M_A$; or when a precision requirement for the gas concentration is high, communication signals (for example, the first communication signal and the second communication signal) of two modules (for example, the module A and the module B) need to be used to calculate the gas concentration $M_{AB}$ according to a differential absorption algorithm.

In this embodiment of this application, a condition in which the gas concentration is calculated based on a sent signal of only one module may be referred to as a general operating condition, and a condition in which the gas concentration is calculated based on synchronously sent signals of two modules is referred to as a warning operating condition. For example, for flammable and explosive gases, users are more concerned that the concentrations of these gases exceed a specific upper limit, which poses a threat to production, life, and personnel health and safety. Therefore, the gas concentration is far lower than a specific warning value, which has a low requirement for measurement precision of a gas concentration. In this way, it is unnecessary to use excessive resources to monitor the gas concentration. Therefore, generally, when the gas concentration is low, fewer resources are used to monitor the gas concentration, and the system is in the general operating condition. When the gas concentration is high, more resources are used to improve the precision of monitoring the gas concentration, and the system is in the warning operating condition. Optionally, in some special scenarios, there is an exact opposite requirement, that is, more resources need to be scheduled to measure the gas concentration only when the gas concentration is lower than a specific warning value. For a scenario in which more resources need to be scheduled to measure the gas concentration only when the gas concentration is lower than a specific warning value, only the determining condition in the embodiment needs to be changed to an opposite meaning. For ease of understanding, in embodiments of this application, an example in which more resources need to be scheduled to measure the gas concentration only when the gas concentration is higher than a specific warning value is mainly used for description.

Optionally, in some feasible implementations, after the second communication device receives the first communication signal and the second communication signal from the first communication device, the second communication device may further determine, based on the first concentration and/or the second concentration estimated in this period of time, whether to send second indication information to the first communication device, where the second indication information indicates the first communication device to stop sending the second communication signal to the second communication device or reduce a quantity of sending times of sending the second communication signal to the second communication device within unit time. For example,

1. When the first concentration is less than a first concentration threshold, the second communication device sends the second indication information to the first communication device;
2. When the second concentration is less than a first concentration threshold, the second communication device sends the second indication information to the first communication device; or
3. When a concentration difference between the first concentration and the second concentration is less than or equal to a second concentration threshold, the second communication device sends the second indication information to the first communication device. Optionally, $\varepsilon = \dfrac{|M_{\{A,\ B\}} - M_{\{A\}}|}{M_{\{A,\ B\}}} \times 100\%$ may also be defined. When $\varepsilon$ is less than or equal to a preset percentage, the second communication device sends the second indication information to the first communication device.

For ease of understanding, an example in which the second communication device sends the second indication information to the first communication device is mainly used for description subsequently. Correspondingly, the first communication device receives the second indication information from the second communication device, and may stop, based on the second indication information, sending the second communication signal to the second communication device or reduce the quantity of sending times of sending the second communication signal to the second communication device within the unit time.

It should be noted that the second indication information may indicate, in a direct or indirect manner, the first communication device to stop sending the second communication signal to the second communication device or reduce the quantity of sending times of sending the second communication signal to the second communication device within the unit time.

For example, when the indication is performed in a direct manner, the second indication information may directly indicate the first communication device to stop sending the second communication signal to the second communication device or reduce the quantity of sending times of sending the second communication signal to the second communication device within the unit time. For example, when the second indication information is 1, the second indication information indicates the first communication device to stop sending the second communication signal to the second communication device; when the second indication information is 0, the second indication information indicates the first communication device to reduce the quantity of sending times of sending the second communication signal to the second communication device within the unit time. Alternatively, when the second indication information is 0, the second indication information indicates the first communication device to reduce the quantity of sending times of sending the second communication signal to the second communication device within the unit time, and when the second indication information is 1, the second indication information indicates the first communication device to stop sending the second communication signal to the second communication device.

For another example, when the indication is performed in an indirect manner, the second indication information may directly include the first concentration and/or the second concentration. Therefore, after the first communication device receives the first concentration and/or the second concentration, the first communication device needs to determine, based on the received first concentration and/or second concentration, whether to stop sending the second communication signal to the second communication device or reduce the quantity of sending times of sending the second communication signal to the second communication device within the unit time. It should be noted that, when the second indication information directly includes the first concentration and/or the second concentration, regardless of a value of the first concentration and/or the second concentration, the second indication information needs to be sent to the first communication device, in other words, when the second indication information includes the first concentration and/or the second concentration, the second communication device does not need to determine, based on the first concentration and/or the second concentration, whether to send the second indication information to the first communication device, instead, the second indication information is sent to the first communication device in real time or periodically.

Generally, when the second indication information includes the first concentration and/or the second concentration, the first communication device determines, based on one of the following, to stop sending the second communication signal to the second communication device or reduce a quantity of sending times of sending the second communication signal to the second communication device within unit time.

1. When the first concentration is less than a first concentration threshold, the first communication device determines to stop sending the second communication signal to the second communication device or reduce the quantity of sending times of sending the second communication signal to the second communication device within the unit time;

2. When the second concentration is less than a first concentration threshold, the first communication device determines to stop sending the second communication signal to the second communication device or reduce the quantity of sending times of sending the second communication signal to the second communication device within the unit time; or

3. When a concentration difference between the first concentration and the second concentration is less than or equal to a second concentration threshold, the first communication device determines to stop sending the second communication signal to the second communication device or reduce the quantity of sending times of sending the second communication signal to the second communication device within the unit time. Optionally,

$$\varepsilon = \frac{|M_{\{A, B\}} - M_{\{A\}}|}{M_{\{A, B\}}} \times 100\%$$

may also be defined. When $\varepsilon$ is less than or equal to a preset percentage, the first communication device determines to stop sending the second communication signal to the second communication device or reduce the quantity of sending times of sending the second communication signal to the second communication device within the unit time.

Optionally, the first communication device may also determine, based on one of the following, not to stop sending the second communication signal to the second communication device or not to reduce a quantity of sending times of sending the second communication signal to the second communication device within unit time.

1. When the first concentration is greater than or equal to a first concentration threshold, the first communication device determines not to stop sending the second communication signal to the second communication device or not to reduce the quantity of sending times of sending the second communication signal to the second communication device within the unit time;

2. When the second concentration is greater than or equal to a first concentration threshold, the first communication device determines not to stop sending the second communication signal to the second communication device or not to reduce the quantity of sending times of sending the second communication signal to the second communication device within the unit time; or

3. When a concentration difference between the first concentration and the second concentration is greater than a second concentration threshold, the first communication device determines not to stop sending the second communication signal to the second communication device or not to reduce the quantity of sending times of sending the second communication signal to the second communication device within the unit time.

It should be noted that, the stopping sending the second communication signal to the second communication device may be understood as disabling the module B, that is, not sending the second communication signal, or that the quantity of sending times of sending the second communication signal to the second communication device within the unit time is 0.

It should be noted that, the reducing the quantity of sending times of sending the second communication signal to the second communication device within the unit time may be understood as not completely disabling the module B, but different from continuous operating of the module A. The module B operates intermittently, that is, within the same unit time, the quantity of times of sending the second communication signal by the module B at the frequency $f_2$ is apparently less than the quantity of times of sending the first communication signal by the module A at the frequency $f_1$. For example, the module A sends a signal whose carrier frequency is $f_1$ at a frequency of once every minute to calculate the gas concentration, and the module B sends a signal whose carrier frequency is $f_2$ at a frequency of once every 10 minutes to calculate the gas concentration.

It should be noted that, the not stopping sending the second communication signal to the second communication device or not reducing the quantity of sending times of sending the second communication signal to the second communication device within the unit time may be understood as that the module B continuously sends the second communication signal to the second communication device, that is, the module A and the module B synchronously send the corresponding communication signal.

It may be understood that, in a general operating condition, when only the module A sends the communication signal but the module B does not send the communication signal, or when the module A sends the communication signal but the module B slows down sending of the communication signal compared with the module A, only the communication signals sent by the module A may be used for calculating $M_{\{A\}}$ separately, and optionally, the signal data last updated by the module B at a previous moment and the communication signal that is of the module A and that is updated in real time may be used for calculating $M'_{\{A,\ B\}}$ together. For example, the module A sends signals whose carrier frequencies are $f_1$ at moments 08:10, 08:11, 08:12, and 08:13, and the module B sends a signal whose carrier frequency is $f_2$ only at 08:10. At the moments 08:11, 08:12, and 08:13, because the module B does not send the signal whose carrier frequency is $f_2$, the communication signal sent by the module B at the moment 08:10 may be used for calculating $M'_{\{A,\ B\}}$. Generally, when $M_{\{A\}}$ or $M'_{\{A,\ B\}}$ is greater than the first concentration threshold, the warning operating condition needs to be entered. Therefore, the module B may be controlled to increase the quantity of times of sending the communication signal, so that the module B and the module A synchronously send the communication signal. In this way, subsequently, communication signals synchronously sent by the modules A and B may be used for calculating $M_{\{A,B\}}$ together. Optionally, in the warning operating condition, only the communication signals sent by the module A may be used for calculating $M_{\{A\}}$ separately. For example, at the moment when the module A and the module B synchronously send the communication signal, the two signals whose the carrier frequencies are $f_1$ and $f_2$ are sent by the module A and the module B may be used for calculating $M_{\{A,B\}}$, and only the communication signals sent by the module A may be used for calculating $M_{\{A\}}$ separately. Therefore, when the calculated $M_{\{A,\ B\}}$ or $M_{\{A\}}$ is less than the first concentration threshold or the concentration difference between $M_{\{A,\ B\}}$ and $M_{\{A\}}$ is less than or equal to the second concentration threshold in the warning operating condition, the second indication information may be sent to control the module B to stop operating or restore to an intermittent operating state, and this cycle begins. In other words, when the gas concentration is low, the module B participates in gas concentration calculation with reduced workloads and resources; when the gas concentration is high, the module B and the module A have the same participation, and the entire system participates in gas concentration estimation with a large workload. This improves precision and accuracy of gas measurement.

In this embodiment of this application, the first communication device is a transmit end of a communication signal, and the second communication device is a receive end of the communication signal. The second communication device calculates the gas concentration based on transmit power of the transmit end for sending the communication signal and receive power of the receive end for receiving the communication signal, so that a gas concentration can be estimated, thereby reducing costs of estimating the gas concentration. In addition, the first communication device may further determine, based on an indication (for example, the first indication information or the second indication information) of the second communication device, to enable or disable the module B. This not only considers a requirement for calculation precision of a gas concentration, but also helps save resources.

The following describes in detail communication apparatuses provided in this application with reference to FIG. 4 to FIG. 7.

FIG. 4 is a diagram of a structure of a communication apparatus according to an embodiment of this application. The communication apparatus shown in FIG. 4 may be configured to perform some or all functions of the first communication device in the method embodiments described in FIG. 2 or FIG. 3. The apparatus may be the first communication device, or may be an apparatus in the first communication device, or an apparatus that can be used together with the first communication device. The communication apparatus may alternatively be a chip system. The communication apparatus shown in FIG. 4 may include a transceiver unit 401 and a processing unit 402. The processing unit 402 is configured to perform data processing, for example, calculate a concentration and determine, based on the concentration, whether to send a second communication signal. The transceiver unit 401 is integrated with a receiving unit and a sending unit. The transceiver unit 401 may also be referred to as a communication unit. Alternatively, the transceiver unit 401 may be split into a receiving unit and a sending unit. The following processing unit 402 and transceiver unit 401 are similar thereto. Details are not described below.

In a first feasible implementation,

the transceiver unit 401 is configured to send a first communication signal to a second communication device;
the transceiver unit 401 is configured to receive a first echo signal of the first communication signal, where a frequency of the first communication signal is $f_1$, and $f_1$ is used for a first gas;
a processing unit 402, configured to determine a first concentration of the first gas based on transmit power of the first communication signal and receive power of the first echo signal; and
the processing unit 402 is configured to determine, based on the first concentration, whether to send a second communication signal to the second communication device.

In a possible implementation, when performing the determining, based on the first concentration, whether to send the second communication signal to the second communication device, the processing unit is 402 configured to: determine, based on one of the following, to send the second communication signal to the second communication device:

when the first concentration is greater than or equal to a first concentration threshold, the second communication signal is determined to be sent to the second communication device; or
when the first concentration is less than a first concentration threshold and the second communication signal is not sent to the second communication device within preset duration, the second communication signal is determined to be sent to the second communication device.

In a possible implementation, when performing the determining, based on the first concentration, whether to send the second communication signal to the second communication device, the processing unit is 402 configured to: determine, based on the following condition, not to send the second communication signal to the second communication device:
when the first concentration is less than a first concentration threshold, the second communication signal is determined not to be sent to the second communication device.

In a possible implementation, the transceiver unit 401 is configured to send the first communication signal and the second communication signal to the second communication device;

the transceiver unit 401 is configured to receive the first echo signal of the first communication signal and a second echo signal of the second communication signal, where a frequency of the second communication signal is $f_2$, $f_2$ is used for the first gas, and an absorption coefficient of the first gas at the frequency of $f_1$ is greater than an absorption coefficient of the first gas at the frequency of $f_2$; and
the processing unit 402 is configured to determine a second concentration of the first gas based on the transmit power of the first communication signal, the receive power of the first echo signal, transmit power of the second communication signal, and receive power of the second echo signal.

In a possible implementation, the processing unit 402 is further configured to:

when the first concentration is less than the first concentration threshold, stop sending the second communication signal to the second communication device or reduce a quantity of sending times of sending the second communication signal to the second communication device within unit time; or
when the second concentration is less than the first concentration threshold, stop sending the second communication signal to the second communication device or reduce a quantity of sending times of sending the second communication signal to the second communication device within unit time.

In a possible implementation, the processing unit 402 is further configured to:
when a concentration difference between the first concentration and the second concentration is less than or equal to a second concentration threshold, stop sending the second communication signal to the second communication device or reduce a quantity of sending times of sending the second communication signal to the second communication device within unit time.

In a possible implementation, a quantity of sending times of the first communication signal within the unit time is greater than or equal to the quantity of sending times of the second communication signal within the unit time.

In a possible implementation, when performing the determining the first concentration of the first gas based on the transmit power of the first communication signal and the receive power of the first echo signal, the processing unit 402 is configured to:
determine the first concentration of the first gas based on the transmit power of the first communication signal, the receive power of the first echo signal, a distance between the first communication device and the second communication device, and the absorption coefficient of the first gas at the frequency of $f_1$.

In a possible implementation, the first concentration satisfies:

$$M_A = \frac{1}{2L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

In a possible implementation, when performing the determining the second concentration of the first gas based on the transmit power of the first communication signal, the receive power of the first echo signal, the transmit power of the second communication signal, and the receive power of the second echo signal, the processing unit is 402 configured to: determine the second concentration of the first gas on a communication link between the second communication device and the second communication device based on the transmit power of the first communication signal, the receive power of the first echo signal, the transmit power of the second communication signal, the receive power of the second echo signal, a distance between the first communication device and the second communication device, the absorption coefficient of the first gas at the frequency of $f_1$, and the absorption coefficient of the first gas at the frequency of $f_2$.

In a possible implementation, the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first echo signal, $P_{B0}(f_1)$ represents the transmit power of the second communication signal, and $P_A(f_1)$ represents the receive power of the second echo signal.

In a possible implementation, both $f_1$ and $f_2$ belong to a licensed frequency band, or $f_1$ belongs to a licensed frequency band, and $f_2$ belongs to an unlicensed frequency band.

It should be noted that, in the first feasible implementation, the transceiver unit 401 may include two modules: a module A and a module B. The module A is configured to send the first communication signal to the second communication device, and receive the first echo signal from the second communication device. The module B is configured to send the second communication signal to the second communication device, and receive the second echo signal from the second communication device. In addition, when the module B is enabled/disabled to operate, if the processing unit 402 determines that the second communication signal needs to be sent to the second communication device, the processing unit 402 may send enabling or disabling indication information to the module B in the transceiver unit 401, where the enabling or disabling indication herein may be an internal implementation of the first communication device. Optionally, the processing unit may also include two modules: a concentration calculation module and a concentration determining module. The concentration calculation module is configured to calculate the first concentration and/or the second concentration, and the concentration determining module is configured to determine to enable or disable the module B.

In a second feasible implementation,

the transceiver unit 401 is configured to send a first communication signal to a second communication device, where a frequency of the first communication signal is $f_1$, $f_1$ is used for a first gas, and transmit power and receive power of the first communication signal are used to determine a first concentration of the first gas; and

the processing unit 402 is configured to determine, based on first indication information from the second communication device, whether to send a second communication signal to the second communication device, where a frequency of the second communication signal is $f_2$, $f_2$ is used for the first gas, and an absorption coefficient of the first gas at the frequency of $f_1$ is greater than an absorption coefficient of the first gas at the frequency of $f_2$.

In a possible implementation, the first indication information indicates the first communication device to send the second communication signal to the second communication device.

In a possible implementation, the first indication information includes the first concentration.

In a possible implementation, the processing unit 402 is further configured to: send the first communication signal and the second communication signal to the second communication device.

In a possible implementation, a quantity of sending times of the first communication signal within unit time is greater than or equal to a quantity of sending times of the second communication signal within the unit time.

In a possible implementation, the processing unit 402 is further configured to: determine, based on second indication information from the second communication device, to stop sending the second communication signal to the second communication device or reduce the quantity of sending times of sending the second communication signal to the second communication device within the unit time.

In a possible implementation, the second indication information indicates to stop sending the second communication signal to the second communication device or reduce the quantity of sending times of sending the second communication signal to the second communication device within the unit time.

In a possible implementation, the second indication information includes the first concentration and/or a second concentration of the first gas, and the second concentration is determined based on the transmit power and the receive power of the first communication signal and transmit power and receive power of the second communication signal.

In a possible implementation, both $f_1$ and $f_2$ belong to a licensed frequency band, or $f_1$ belongs to a licensed frequency band, and $f_2$ belongs to an unlicensed frequency band.

It should be noted that, in the second feasible implementation, the transceiver unit 401 may include two modules: a module A and a module B. The module A is configured to send the first communication signal to the second communication device, and the module B is configured to send the second communication signal to the second communication device. In addition, when the module B is enabled/disabled to operate, if the processing unit 402 determines that the second communication signal needs to be sent to the second communication device, the processing unit 402 may send enabling or disabling indication information to the module B in the transceiver unit 401, where the enabling or disabling indication herein may be an internal implementation of the first communication device.

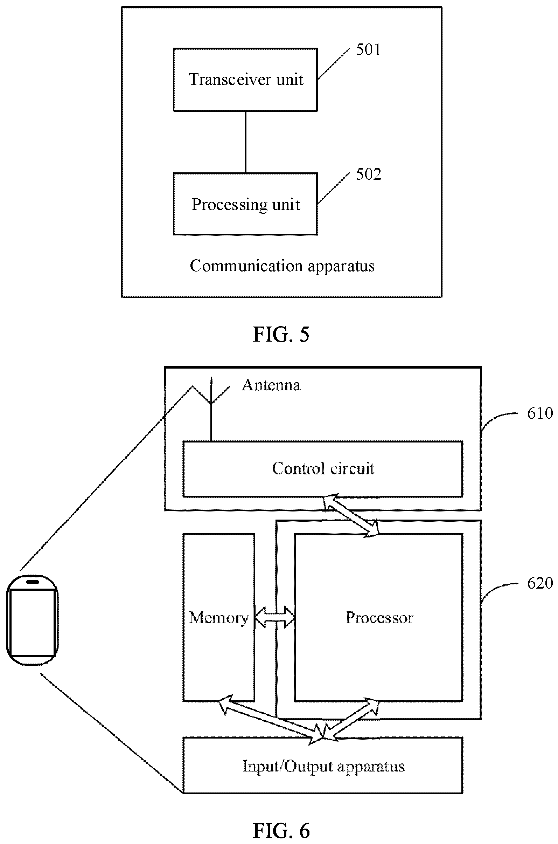

FIG. 5 is a diagram of a structure of another communication apparatus according to an embodiment of this application. The communication apparatus shown in FIG. 5 may be configured to perform some or all functions of the second communication device in the method embodiments described in FIG. 2 or FIG. 3. The apparatus may be the second communication device, or may be an apparatus in the second communication device, or an apparatus that can be used together with the second communication device. The communication apparatus may alternatively be a chip system. The communication apparatus shown in FIG. 5 may include a transceiver unit 501 and a processing unit 502. The processing unit 502 is configured to perform data processing, for example, calculate a concentration and determine, based on the concentration, whether to send a second communication signal. The transceiver unit 501 is integrated with a receiving unit and a sending unit. The transceiver unit 501 may also be referred to as a communication unit. Alternatively, the transceiver unit 501 may be split into a receiving unit and a sending unit. The following processing unit 502 and transceiver unit 501 are similar thereto. Details are not described below.

The transceiver unit 501 is configured to receive a first communication signal from a first communication device, where a frequency of the first communication signal is $f_1$, and $f_1$ is used for a first gas; and

the processing unit 502 is configured to determine a first concentration of the first gas based on receive power and transmit power of the first communication signal.

The processing unit 502 is configured to determine, based on the first concentration, whether to send first indication information to the first communication device, where the first indication information is used to determine that the first communication device sends a second communication signal to the second communication device; or

the transceiver unit 501 is configured to send first indication information to the first communication device, where the first indication information is used to determine whether the first communication device sends a second communication signal to the second communication device, and the first indication information includes the first concentration.

In a possible implementation, when performing the determining, based on the first concentration, whether to send the first indication information to the first communication device, the processing unit 502 is configured to:

determine, based on one of the following, to send the first indication information to the first communication device:

when the first concentration is greater than or equal to a first concentration threshold, the first indication information is determined to be sent to the first communication device; or

when the first concentration is less than a first concentration threshold, and the first indication information is not sent to the first communication device within preset duration, the first indication information is determined to be sent to the first communication device.

In a possible implementation, when performing the determining, based on the first concentration, whether to send the first indication information to the first communication device, the processing unit 502 is configured to:

determine, based on the following condition, not to send the first indication information to the first communication device: when the first concentration is less than a first concentration threshold, the first indication information is determined not to be sent to the first communication device.

In a possible implementation, the first indication information is used to determine that the first communication device sends the second communication signal to the second communication device;

the transceiver unit 501 is further configured to receive the first communication signal and the second communication signal from the first communication device; and

the processing unit 502 is further configured to determine a second concentration of the first gas based on the transmit power and the receive power of the first communication signal and transmit power and receive power of the second communication signal.

In a possible implementation, the processing unit 502 is further configured to:

when the first concentration is less than the first concentration threshold, send second indication information to the first communication device, where the second indication information indicates the first communication device to stop sending the second communication signal to the second communication device or reduce a quantity of sending times of sending the second communication signal to the second communication device within unit time; or

when the second concentration is less than the first concentration threshold, send second indication information to the first communication device, where the second indication information indicates the first communication device to stop sending the second communication signal to the second communication device or reduce a quantity of sending times of sending the second communication signal to the second communication device within unit time.

In a possible implementation, the processing unit 502 is further configured to:
when a concentration difference between the first concentration and the second concentration is less than or equal to a second concentration threshold, send second indication information to the first communication device, where the second indication information indicates the first communication device to stop sending the second communication signal to the second communication device or reduce a quantity of sending times of sending the second communication signal to the second communication device within unit time.

In a possible implementation, the processing unit 502 is further configured to:
send second indication information to the first communication device, where the second indication information includes the first concentration and/or the second concentration.

In a possible implementation, when performing the determining the first concentration of the first gas based on the receive power and the transmit power of the first communication signal, the processing unit 502 is configured to:
determine the first concentration of the first gas based on the transmit power of the first communication signal, the receive power of the first communication signal, a distance between the second communication device and the first communication device, and an absorption coefficient of the first gas at the frequency of $f_1$.

In a possible implementation, the first concentration satisfies:

$$M_A = \frac{1}{L \cdot \sigma(f_1)} \ln \frac{P_{A0}(f_1)}{P_A(f_1)},$$

where $M_A$ represents the first concentration, $\sigma(f_1)$ represents the absorption coefficient of the first gas at the frequency of $f_1$, $L$ represents the distance between the second communication device and the first communication device, $P_{A0}(f_1)$ represents the transmit power of the first communication signal, and $P_A(f_1)$ represents the receive power of the first communication signal.

In a possible implementation, when performing the determining the second concentration of the first gas based on the transmit power and the receive power of the first communication signal and the transmit power and the receive power of the second communication signal, the processing unit 502 is configured to:
determine the second concentration of the first gas on a communication link between the first communication device and the first communication device based on the transmit power of the first communication signal, the receive power of the first communication signal, the transmit power of the second communication signal, the receive power of the second communication signal, a distance between the second communication device and the first communication device, an absorption coefficient of the first gas at the frequency of $f_1$, and an absorption coefficient of the first gas at a frequency of $f_2$.

In a possible implementation, the second concentration satisfies:

$$M_{AB} = \frac{1}{\Delta L [\sigma(f_1) - \sigma(f_2)]} \ln \frac{P_B(f_2) \cdot P_{A0}(f_1)}{P_A(f_1) \cdot P_{B0}(f_2)},$$

where $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the second communication device and the first communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

In a possible implementation, both $f_1$ and $f_2$ belong to a licensed frequency band, or $f_1$ belongs to a licensed frequency band, and $f_2$ belongs to an unlicensed frequency band.

It should be noted that the processing unit may also include two modules: a concentration calculation module and a concentration determining module. The concentration calculation module is configured to calculate the first concentration and/or the second concentration, and the concentration determining module is configured to determine to enable or disable the module B.

For another possible implementation of the communication apparatus, refer to related descriptions of the first communication device and the second communication device in the method embodiments corresponding to FIG. 2 and FIG. 3. Details are not described herein.

FIG. 6 is a diagram of a structure of another communication apparatus according to an embodiment of this application. As shown in FIG. 6, the communication apparatus may be the first communication device or the second communication device described in embodiments of this application. The first communication device or the second communication device may be specifically a terminal device. For ease of description, FIG. 6 shows only main components of the terminal device. As shown in FIG. 6, the terminal device includes a processor, a memory, a control circuit, an antenna, and an input/output apparatus. The processor is mainly configured to: process a communication protocol and communication data, control the entire terminal device, execute a software program, and process data of the software program. The memory is mainly configured to store the software program and data. The control circuit is mainly configured to: convert a baseband signal and a radio frequency signal, and process the radio frequency signal. The antenna is mainly configured to receive or send a radio frequency signal in a form of an electromagnetic wave. The input/output apparatus, for example, a touchscreen, a display, a microphone, or a keyboard, is mainly configured to receive data input by a user and output data to the user.

For example, the terminal device is a mobile phone. After the terminal device is powered on, the processor may read a software program in a storage unit, interpret and execute instructions of the software program, and process data of the software program. When data needs to be sent wirelessly, the processor performs baseband processing on the to-be-sent data, and then outputs a baseband signal to the control circuit. The control circuit performs radio frequency processing on the baseband signal, and then sends a radio frequency signal to the outside in the form of the electromagnetic wave through the antenna. When data is sent to the terminal device, the control circuit receives a radio frequency signal through the antenna, converts the radio frequency signal into a baseband signal, and outputs the baseband signal to the processor. The processor converts the baseband signal into data, and processes the data.

A person skilled in the art may understand that, for ease of description, FIG. 6 shows only one memory and only one processor. In some embodiments, the terminal device may include a plurality of processors and memories. The memory may also be referred to as a storage medium, a storage device, or the like. This is not limited in embodiments of this application.

In an optional implementation, the processor may include a baseband processor and a central processing unit. The baseband processor is mainly configured to process the communication protocol and the communication data. The central processing unit is mainly configured to: control the entire terminal device, execute the software program, and process the data of the software program. The processor in FIG. 6 integrates functions of the baseband processor and the central processing unit. A person skilled in the art may understand that the baseband processor and the central processing unit may alternatively be processors independent of each other, and are interconnected through a technology like a bus. The terminal device may include a plurality of baseband processors to adapt to different network standards. The terminal device may include a plurality of central processing units to enhance a processing capability of the terminal device. Components of the terminal device may be connected through various buses. The baseband processor may alternatively be expressed as a baseband processing circuit or a baseband processing chip. The central processing unit may alternatively be expressed as a central processing circuit or a central processing chip. A function of processing the communication protocol and the communication data may be built in the processor, or may be stored in the storage unit in a form of a software program. The processor executes the software program to implement a baseband processing function.

In an example, the antenna and the control circuit that have receiving or sending functions may be considered as a transceiver unit 610 of the terminal device, and the processor having a processing function may be considered as a processing unit 620 of the terminal device. As shown in FIG. 6, the terminal device includes the transceiver unit 610 and the processing unit 620. The transceiver unit may also be referred to as a transceiver, a transceiver machine, a transceiver apparatus, or the like. Optionally, a component that is in the transceiver unit 610 and that is configured to implement a receiving function may be considered as a receiving unit, and a component that is in the transceiver unit 610 and that is configured to implement a sending function may be considered as a sending unit. In other words, the transceiver unit 610 includes the receiving unit and the sending unit. For example, the receiving unit may also be referred to as a receiver, a receive machine, or a receiving circuit, and the sending unit may also be referred to as a transmitter, a transmit machine, or a transmitting circuit.

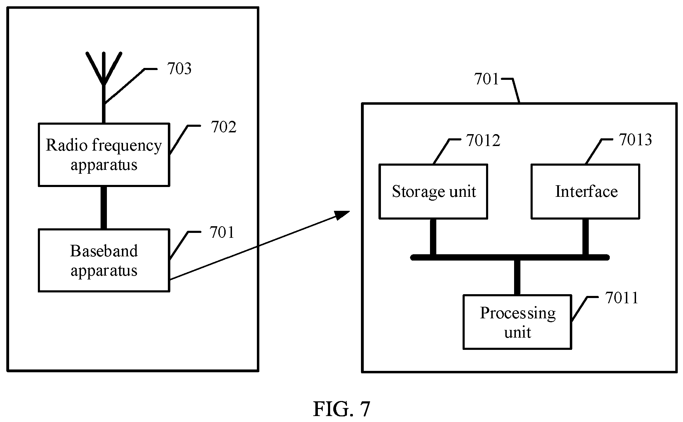

FIG. 7 is a diagram of a structure of another communication apparatus according to an embodiment of this application. As shown in FIG. 7, the communication apparatus may be the first communication device or the second communication device described in embodiments of this application. The first communication device or the second communication device may be specifically an access network device. The access network device includes a baseband apparatus 701, a radio frequency apparatus 702, and an antenna 703. In an uplink direction, the radio frequency apparatus 702 receives, through the antenna 703, information sent by a terminal device, and sends, to the baseband apparatus 701, the information sent by the terminal device for processing. In a downlink direction, the baseband apparatus 701 processes information from the terminal device, and sends processed information to the radio frequency apparatus 702. The radio frequency apparatus 702 processes the information from the terminal device, and then sends processed information to the terminal device through the antenna 703.

The baseband apparatus 701 includes one or more processing units 7011, a storage unit 7012, and an interface 7013. The processing unit 7011 is configured to support the access network device in performing a function of the access network device in the foregoing method embodiments. The storage unit 7012 is configured to store a software program and/or data. The interface 7013 is configured to exchange information with the radio frequency apparatus 702. The interface includes an interface circuit, configured to input and output information. In an implementation, the processing unit is an integrated circuit, for example, one or more application-specific integrated circuits (application-specific integrated circuits, ASICs), one or more digital signal processors (digital signal processors, DSPs), one or more field programmable gate arrays (field programmable gate arrays, FPGAs), or a combination of such integrated circuits. These integrated circuits may be integrated together to form a chip. The storage unit 7012 and the processing unit 7011 may be located in a same chip, that is, an on-chip storage element. Alternatively, the storage unit 7012 and the processing unit 7011 may be located in different chips from the processing unit 7011, that is, an off-chip storage element. The storage unit 7012 may be a memory, or may be a collective term of a plurality of memories or storage elements.

The access network device may implement a part of or all steps in the foregoing method embodiments in a form of scheduling a program by one or more processing units. For example, a corresponding function of the first communication device or the second communication device in FIG. 2 or FIG. 3 is implemented. The one or more processing units may support radio access technologies of a same standard, or may support radio access standards of different standards.

An embodiment of this application further provides a computer-readable storage medium. The computer-readable storage medium stores instructions. When the instructions are run on a processor, method procedures in the foregoing method embodiments are implemented.

An embodiment of this application further provides a computer program product. When the computer program product runs on a processor, method procedures in the foregoing method embodiments are implemented.

A person of ordinary skill in the art may be aware that, with reference to the examples described in embodiments disclosed in this specification, units and steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

In the several embodiments provided in this application, it should be understood that the disclosed system, apparatus, and methods may be implemented in other manners. For example, the described apparatus embodiment is merely an example. For example, division into units is merely logical function division. Units described as separate components may or may not be physically separate, and components displayed as units may or may not be physical units, may be located in one position, or may be distributed on a plurality of network units. A part or all of the units may be selected based on actual requirements to achieve the objectives of the solutions of embodiments.

When the functions are implemented in a form of a software function unit and sold or used as an independent product, the functions may be stored in a computer-readable storage medium. Based on such an understanding, the technical solutions of this application essentially, or the part contributing to the conventional technology, or some of the technical solutions may be implemented in a form of a software product. The computer software product is stored in a storage medium and includes several instructions for instructing a computer device (which may be a personal computer, a server, or a network device) to perform all or some of the steps of the methods described in embodiments of this application. The computer-readable storage medium may be any usable medium that can be accessed by a computer. For example, the computer-readable medium may include but is not limited to: a random access memory (random access memory, RAM), a read-only memory (read-only memory, ROM), a programmable read-only memory (programmable ROM, PROM), an erasable programmable read-only memory (erasable PROM, EPROM), an electrically erasable programmable read-only memory (electrically erasable programmable read-only memory, EEPROM), a compact disc read-only memory (compact disc read-only memory, CD-ROM), a universal serial bus flash disk (universal serial bus flash disk), a removable hard disk, another optical disc memory, a magnetic disk storage medium, or another magnetic storage device, or any other medium that can be used to carry or store expected program code in a form of instructions or a data structure and that can be accessed by a computer. In addition, by way of example rather than limitative description, many forms of RAMs may be used, for example, a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchronous link dynamic random access memory (synchlink DRAM, SLDRAM), and a direct rambus random access memory (direct rambus RAM, DR RAM).

The foregoing descriptions are merely specific implementations of this application, but are not intended to limit the protection scope of embodiments of this application. Any variation or replacement readily figured out by a person skilled in the art within the technical scope disclosed in embodiments of this application shall fall within the protection scope of embodiments of this application. Therefore, the protection scope of embodiments of this application should be subject to the protection scope of the claims.

**Claims**

1.  A method for determining a gas concentration, wherein the method is applied to a first communication device and comprises:

    sending a first communication signal to a second communication device;
    receiving a first echo signal of the first communication signal, wherein a frequency of the first communication signal is $f_1$, and $f_1$ is used for a first gas;
    determining a first concentration of the first gas based on transmit power of the first communication signal and receive power of the first echo signal; and
    determining, based on the first concentration, whether to send a second communication signal to the second communication device.

2.  The method according to claim 1, wherein the determining, based on the first concentration, whether to send a second communication signal to the second communication device comprises:
    determining, based on one of the following, to send the second communication signal to the second communication device:

    when the first concentration is greater than or equal to a first concentration threshold, the second communication signal is determined to be sent to the second communication device; or
    when the first concentration is less than a first concentration threshold and the second communication signal is not sent to the second communication device within preset duration, the second communication signal is determined to be sent to the second communication device.

3.  The method according to claim 1, wherein the determining, based on the first concentration, whether to send a second communication signal to the second communication device comprises:
    determining, based on the following condition, not to send the second communication signal to the second communication device:
    when the first concentration is less than a first concentration threshold, the second communication signal is determined not to be sent to the second communication device.

4.  The method according to claim 2, wherein after the second communication signal is determined, based on the first concentration, to be sent to the second communication device, the method further comprises:

    sending the first communication signal and the second communication signal to the second communication device;
    receiving the first echo signal of the first communication signal and a second echo signal of the second communication signal, wherein a frequency of the second communication signal is $f_2$, $f_2$ is used for the first gas, and an absorption coefficient of the first gas at the frequency of $f_1$ is greater than an absorption coefficient of the first gas at the frequency of $f_2$; and
    determining a second concentration of the first gas based on the transmit power of the first communication signal, the receive power of the first echo signal, transmit power of the second communication signal, and receive power of the second echo signal.

5.  The method according to claim 4, wherein after the sending the first communication signal and the second communication signal to the second communication device, the method further comprises:

    when the first concentration is less than the first concentration threshold, stopping sending the second communication signal to the second communication device or reducing a quantity of sending times of sending the second communication signal to the second communication device within unit time; or
    when the second concentration is less than the first concentration threshold, stopping sending the second communication signal to the second communication device or reducing a quantity of sending times of sending the second communication signal to the second communication device within unit time.

6. The method according to claim 4, wherein after the sending the first communication signal and the second communication signal to the second communication device, the method further comprises:
when a concentration difference between the first concentration and the second concentration is less than or equal to a second concentration threshold, stopping sending the second communication signal to the second communication device or reducing a quantity of sending times of sending the second communication signal to the second communication device within unit time.

7. The method according to any one of claims 4 to 6, wherein a quantity of sending times of the first communication signal within the unit time is greater than or equal to the quantity of sending times of the second communication signal within the unit time.

8. The method according to any one of claims 1 to 7, wherein the determining a first concentration of the first gas based on transmit power of the first communication signal and receive power of the first echo signal comprises:
determining the first concentration of the first gas based on the transmit power of the first communication signal, the receive power of the first echo signal, a distance between the first communication device and the second communication device, and the absorption coefficient of the first gas at the frequency of $f_1$.

9. The method according to claim 8, wherein the first concentration satisfies:

$$M_A = \frac{1}{2L \cdot \sigma(f_1)} \ln \frac{P_{A0}(f_1)}{P_A(f_1)},$$

wherein $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

10. The method according to any one of claims 4 to 7, wherein the determining a second concentration of the first gas based on the transmit power of the first communication signal, the receive power of the first echo signal, transmit power of the second communication signal, and receive power of the second echo signal comprises:
determining the second concentration of the first gas on a communication link between the second communication device and the second communication device based on the transmit power of the first communication signal, the receive power of the first echo signal, the transmit power of the second communication signal, the receive power of the second echo signal, a distance between the first communication device and the second communication device, the absorption coefficient of the first gas at the frequency of $f_1$, and the absorption coefficient of the first gas at the frequency of $f_2$.

11. The method according to claim 10, wherein the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

wherein $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f_1)$ represents the receive power of the first echo signal, $P_{B0}(f_1)$ represents the transmit power of the second communication signal, and $P_A(f_1)$ represents the receive power of the second echo signal.

12. The method according to any one of claims 1 to 11, wherein both $f_1$ and $f_2$ belong to a licensed frequency band, or $f_1$ belongs to a licensed frequency band, and $f_2$ belongs to an unlicensed frequency band.

13. A method for determining a gas concentration, wherein the method is applied to a first communication device and comprises:

sending a first communication signal to a second communication device, wherein a frequency of the first communication signal is $f_1$, $f_1$ is used for a first gas, and transmit power and receive power of the first communication signal are used to determine a first concentration of the first gas; and

determining, based on first indication information from the second communication device, whether to send a second communication signal to the second communication device, wherein a frequency of the second communication signal is $f_2$, $f_2$ is used for the first gas, and an absorption coefficient of the first gas at the frequency of $f_1$ is greater than an absorption coefficient of the first gas at the frequency of $f_2$.

14. The method according to claim 13, wherein the first indication information indicates the first communication device to send the second communication signal to the second communication device.

15. The method according to claim 13, wherein the first indication information comprises the first concentration.

16. The method according to any one of claims 13 to 15, wherein after the second communication signal is determined to be sent to the second communication device based on the first indication information from the second communication device, the method further comprises:
sending the first communication signal and the second communication signal to the second communication device.

17. The method according to claim 16, wherein a quantity of sending times of the first communication signal within unit time is greater than or equal to a quantity of sending times of the second communication signal within the unit time.

18. The method according to claim 16 or 17, wherein after the sending the first communication signal and the second communication signal to the second communication device, the method further comprises:
determining, based on second indication information from the second communication device, to stop sending the second communication signal to the second communication device or reduce the quantity of sending times of sending the second communication signal to the second communication device within the unit time.

19. The method according to claim 18, wherein the second indication information indicates to stop sending the second communication signal to the second communication device or reduce the quantity of sending times of sending the second communication signal to the second communication device within the unit time.

20. The method according to claim 18, wherein the second indication information comprises the first concentration and/or a second concentration of the first gas, and the second concentration is determined based on the transmit power and the receive power of the first communication signal and transmit power and receive power of the second communication signal.

21. The method according to any one of claims 13 to 20, wherein both $f_1$ and $f_2$ belong to a licensed frequency band, or $f_1$ belongs to a licensed frequency band, and $f_2$ belongs to an unlicensed frequency band.

22. A method for determining a gas concentration, wherein the method is applied to a second communication device and comprises:

receiving a first communication signal from a first communication device, wherein a frequency of the first communication signal is $f_1$, and $f_1$ is used for a first gas;

determining a first concentration of the first gas based on receive power and transmit power of the first communication signal; and

determining, based on the first concentration, whether to send first indication information to the first communication device, wherein the first indication information is used to determine that the first communication device sends a second communication signal to the second communication device; or

sending first indication information to the first communication device, wherein the first indication information is used to determine whether the first communication device sends a second communication signal to the second communication device, and the first indication information comprises the first concentration.

23. The method according to claim 22, wherein the determining, based on the first concentration, whether to send first indication information to the first communication device comprises:
determining, based on one of the following, to send the first indication information to the first communication device:

when the first concentration is greater than or equal to a first concentration threshold, the first indication information is determined to be sent to the first communication device; or

when the first concentration is less than a first concentration threshold, and the first indication information is not sent to the first communication device within preset duration, the first indication information is determined to be sent to the first communication device.

24. The method according to claim 22, wherein the determining, based on the first concentration, whether to send first indication information to the first communication device comprises:

determining, based on the following condition, not to send the first indication information to the first communication device:

when the first concentration is less than a first concentration threshold, the first indication information is determined not to be sent to the first communication device.

25. The method according to claim 22 or 23, wherein the first indication information is used to determine that the first communication device sends the second communication signal to the second communication device; and the method further comprises:

receiving the first communication signal and the second communication signal from the first communication device; and

determining a second concentration of the first gas based on the transmit power and the receive power of the first communication signal and transmit power and receive power of the second communication signal.

26. The method according to claim 25, wherein after the receiving the first communication signal and the second communication signal from the first communication device, the method further comprises:

when the first concentration is less than the first concentration threshold, sending second indication information to the first communication device, wherein the second indication information indicates the first communication device to stop sending the second communication signal to the second communication device or reduce a quantity of sending times of sending the second communication signal to the second communication device within unit time; or

when the second concentration is less than the first concentration threshold, sending second indication information to the first communication device, wherein the second indication information indicates the first communication device to stop sending the second communication signal to the second communication device or reduce a quantity of sending times of sending the second communication signal to the second communication device within unit time.

27. The method according to claim 25, wherein after the receiving the first communication signal and the second communication signal from the first communication device, the method further comprises:

when a concentration difference between the first concentration and the second concentration is less than or equal to a second concentration threshold, sending second indication information to the first communication device, wherein the second indication information indicates the first communication device to stop sending the second communication signal to the second communication device or reduce a quantity of sending times of sending the second communication signal to the second communication device within unit time.

28. The method according to claim 25, wherein after the receiving the first communication signal and the second communication signal from the first communication device, the method further comprises:

sending second indication information to the first communication device, wherein the second indication information comprises the first concentration and/or the second concentration.

29. The method according to any one of claims 22 to 28, wherein the determining a first concentration of the first gas based on receive power and transmit power of the first communication signal comprises:

determining the first concentration of the first gas based on the transmit power of the first communication signal, the receive power of the first communication signal, a distance between the second communication device and the first communication device, and an absorption coefficient of the first gas at the frequency of $f_1$.

30. The method according to claim 29, wherein the first concentration satisfies:

$$M_A = \frac{1}{L \cdot \sigma(f_1)} \ln \frac{P_{A0}(f_1)}{P_A(f_1)},$$

wherein $M_A$ represents the first concentration, $\sigma(f_1)$ represents the absorption coefficient of the first gas at the frequency of $f_1$, $L$ represents the distance between the second communication device and the first communication device, $P_{A0}(f_1)$ represents the transmit power of the first communication signal, and $P_A(f_1)$ represents the receive power of the first communication signal.

31. The method according to any one of claims 25 to 28, wherein the determining a second concentration of the first gas based on the transmit power and the receive power of the first communication signal and transmit power and receive power of the second communication signal comprises:
determining the second concentration of the first gas on a communication link between the first communication device and the first communication device based on the transmit power of the first communication signal, the receive power of the first communication signal, the transmit power of the second communication signal, the receive power of the second communication signal, a distance between the second communication device and the first communication device, an absorption coefficient of the first gas at the frequency of $f_1$, and an absorption coefficient of the first gas at a frequency of $f_2$.

32. The method according to claim 31, wherein the second concentration satisfies:

$$M_{AB} = \frac{1}{\Delta L[\sigma(f_1) - \sigma(f_2)]} \ln \frac{P_B(f_2) \cdot P_{A0}(f_1)}{P_A(f_1) \cdot P_{B0}(f_2)},$$

wherein $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the second communication device and the first communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

33. The method according to any one of claims 22 to 32, wherein both $f_1$ and $f_2$ belong to a licensed frequency band, or $f_1$ belongs to a licensed frequency band, and $f_2$ belongs to an unlicensed frequency band.

34. An apparatus for determining a gas concentration, wherein the apparatus is a first communication device and comprises:

a transceiver unit, configured to send a first communication signal to a second communication device, wherein the transceiver unit is configured to receive a first echo signal of the first communication signal, wherein a frequency of the first communication signal is $f_1$, and $f_1$ is used for a first gas; and
a processing unit, configured to determine a first concentration of the first gas based on transmit power of the first communication signal and receive power of the first echo signal, wherein
the processing unit is configured to determine, based on the first concentration, whether to send a second communication signal to the second communication device.

35. The apparatus according to claim 34, wherein when performing the determining, based on the first concentration, whether to send the second communication signal to the second communication device, the processing unit is configured to:
determine, based on one of the following, to send the second communication signal to the second communication device:

when the first concentration is greater than or equal to a first concentration threshold, the second communication signal is determined to be sent to the second communication device; or
when the first concentration is less than a first concentration threshold and the second communication signal is not sent to the second communication device within preset duration, the second communication signal is determined to be sent to the second communication device.

**36.** The apparatus according to claim 34, wherein when performing the determining, based on the first concentration, whether to send the second communication signal to the second communication device, the processing unit is configured to:
determine, based on the following condition, not to send the second communication signal to the second communication device:
when the first concentration is less than a first concentration threshold, the second communication signal is determined not to be sent to the second communication device.

**37.** The apparatus according to claim 35, wherein

the transceiver unit is configured to send the first communication signal and the second communication signal to the second communication device;
the transceiver unit is configured to receive the first echo signal of the first communication signal and a second echo signal of the second communication signal, wherein a frequency of the second communication signal is $f_2$, $f_2$ is used for the first gas, and an absorption coefficient of the first gas at the frequency of $f_1$ is greater than an absorption coefficient of the first gas at the frequency of $f_2$; and
the processing unit is configured to determine a second concentration of the first gas based on the transmit power of the first communication signal, the receive power of the first echo signal, transmit power of the second communication signal, and receive power of the second echo signal.

**38.** The apparatus according to claim 37, wherein the processing unit is further configured to:

when the first concentration is less than the first concentration threshold, stop sending the second communication signal to the second communication device or reduce a quantity of sending times of sending the second communication signal to the second communication device within unit time; or
when the second concentration is less than the first concentration threshold, stop sending the second communication signal to the second communication device or reduce a quantity of sending times of sending the second communication signal to the second communication device within unit time.

**39.** The apparatus according to claim 37, wherein the processing unit is further configured to:
when a concentration difference between the first concentration and the second concentration is less than or equal to a second concentration threshold, stop sending the second communication signal to the second communication device or reduce a quantity of sending times of sending the second communication signal to the second communication device within unit time.

**40.** The apparatus according to any one of claims 37 to 39, wherein a quantity of sending times of the first communication signal within the unit time is greater than or equal to the quantity of sending times of the second communication signal within the unit time.

**41.** The apparatus according to any one of claims 34 to 40, wherein when performing the determining the first concentration of the first gas based on the transmit power of the first communication signal and the receive power of the first echo signal, the processing unit is configured to:
determine the first concentration of the first gas based on the transmit power of the first communication signal, the receive power of the first echo signal, a distance between the first communication device and the second communication device, and the absorption coefficient of the first gas at the frequency of $f_1$.

**42.** The apparatus according to claim 41, wherein the first concentration satisfies:

$$M_A = \frac{1}{2L \cdot \sigma(f_1)} \ln \frac{P_{A0}(f_1)}{P_A(f_1)},$$

wherein $M_A$ represents the first concentration, $\sigma(f_1)$ represents the absorption coefficient of the first gas at the frequency of $f_1$, $L$ represents the distance between the first communication device and the second communication device, $P_{A0}(f_1)$ represents the transmit power of the first communication signal, and $P_A(f_1)$ represents the receive power of the first echo signal.

**43.** The apparatus according to any one of claims 37 to 40, wherein when performing the determining the second concentration of the first gas based on the transmit power of the first communication signal, the receive power of the first echo signal, the transmit power of the second communication signal, and the receive power of the second echo signal, the processing unit is configured to:

determine the second concentration of the first gas on a communication link between the second communication device and the second communication device based on the transmit power of the first communication signal, the receive power of the first echo signal, the transmit power of the second communication signal, the receive power of the second echo signal, a distance between the first communication device and the second communication device, the absorption coefficient of the first gas at the frequency of $f_1$, and the absorption coefficient of the first gas at the frequency of $f_2$.

44. The apparatus according to claim 43, wherein the second concentration satisfies:

$$M_{AB} = \frac{1}{2\Delta L[\sigma(f_1) - \sigma(f_2)]} \ln \frac{P_B(f_2) \cdot P_{A0}(f_1)}{P_A(f_1) \cdot P_{B0}(f_2)},$$

wherein $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the first communication device and the second communication device, $P_{A0}(f_1)$ represents the transmit power of the first communication signal, $P_A(f)$ represents the receive power of the first echo signal, $P_{B0}(f_1)$ represents the transmit power of the second communication signal, and $P_A(f_1)$ represents the receive power of the second echo signal.

45. The apparatus according to any one of claims 34 to 44, wherein both $f_1$ and $f_2$ belong to a licensed frequency band, or $f_1$ belongs to a licensed frequency band, and $f_2$ belongs to an unlicensed frequency band.

46. An apparatus for determining a gas concentration, wherein the apparatus is a first communication device and comprises:

a transceiver unit, configured to send a first communication signal to a second communication device, wherein a frequency of the first communication signal is $f_1$, $f_1$ is used for a first gas, and transmit power and receive power of the first communication signal are used to determine a first concentration of the first gas; and
a processing unit, configured to determine, based on first indication information from the second communication device, whether to send a second communication signal to the second communication device, wherein a frequency of the second communication signal is $f_2$, $f_2$ is used for the first gas, and an absorption coefficient of the first gas at the frequency of $f_1$ is greater than an absorption coefficient of the first gas at the frequency of $f_2$.

47. The apparatus according to claim 46, wherein the first indication information indicates the first communication device to send the second communication signal to the second communication device.

48. The apparatus according to claim 46, wherein the first indication information comprises the first concentration.

49. The apparatus according to any one of claims 46 to 48, wherein the processing unit is further configured to:
send the first communication signal and the second communication signal to the second communication device.

50. The apparatus according to claim 49, wherein a quantity of sending times of the first communication signal within unit time is greater than or equal to a quantity of sending times of the second communication signal within the unit time.

51. The apparatus according to claim 49 or 50, wherein the processing unit is further configured to:
determine, based on second indication information from the second communication device, to stop sending the second communication signal to the second communication device or reduce the quantity of sending times of sending the second communication signal to the second communication device within the unit time.

52. The apparatus according to claim 51, wherein the second indication information indicates to stop sending the second communication signal to the second communication device or reduce the quantity of sending times of sending the second communication signal to the second communication device within the unit time.

53. The apparatus according to claim 51, wherein the second indication information comprises the first concentration and/or a second concentration of the first gas, and the second concentration is determined based on the transmit power and the receive power of the first communication signal and transmit power and receive power of the second communication signal.

54. The apparatus according to any one of claims 46 to 53, wherein both $f_1$ and $f_2$ belong to a licensed frequency band, or $f_1$ belongs to a licensed frequency band, and $f_2$ belongs to an unlicensed frequency band.

55. An apparatus for determining a gas concentration, wherein the apparatus is a second communication device and comprises:

a transceiver unit, configured to receive a first communication signal from a first communication device, wherein a frequency of the first communication signal is $f_1$, and $f_1$ is used for a first gas; and
a processing unit, configured to determine a first concentration of the first gas based on receive power and transmit power of the first communication signal, wherein
the processing unit is configured to determine, based on the first concentration, whether to send first indication information to the first communication device, wherein the first indication information is used to determine that the first communication device sends a second communication signal to the second communication device; or
the transceiver unit is configured to send first indication information to the first communication device, wherein the first indication information is used to determine whether the first communication device sends a second communication signal to the second communication device, and the first indication information comprises the first concentration.

56. The apparatus according to claim 55, wherein when performing the determining, based on the first concentration, whether to send the first indication information to the first communication device, the processing unit is configured to: determine, based on one of the following, to send the first indication information to the first communication device:

when the first concentration is greater than or equal to a first concentration threshold, the first indication information is determined to be sent to the first communication device; or
when the first concentration is less than a first concentration threshold, and the first indication information is not sent to the first communication device within preset duration, the first indication information is determined to be sent to the first communication device.

57. The apparatus according to claim 55, wherein when performing the determining, based on the first concentration, whether to send the first indication information to the first communication device, the processing unit is configured to: determine, based on the following condition, not to send the first indication information to the first communication device:
when the first concentration is less than a first concentration threshold, the first indication information is determined not to be sent to the first communication device.

58. The apparatus according to claim 55 or 56, wherein the first indication information is used to determine that the first communication device sends the second communication signal to the second communication device;

the transceiver unit is further configured to receive the first communication signal and the second communication signal from the first communication device; and
the processing unit is further configured to determine a second concentration of the first gas based on the transmit power and the receive power of the first communication signal and transmit power and receive power of the second communication signal.

59. The apparatus according to claim 58, wherein the processing unit is further configured to:

when the first concentration is less than the first concentration threshold, send second indication information to the first communication device, wherein the second indication information indicates the first communication device to stop sending the second communication signal to the second communication device or reduce a quantity of sending times of sending the second communication signal to the second communication device within unit time; or
when the second concentration is less than the first concentration threshold, send second indication information to the first communication device, wherein the second indication information indicates the first communication device to stop sending the second communication signal to the second communication device or reduce a quantity of sending times of sending the second communication signal to the second communication device within unit time.

60. The apparatus according to claim 58, wherein the processing unit is further configured to:
when a concentration difference between the first concentration and the second concentration is less than or equal to a second concentration threshold, send second indication information to the first communication device, wherein the second indication information indicates the first communication device to stop sending the second communication signal to the second communication device or reduce a quantity of sending times of sending the second communication signal to the second communication device within unit time.

61. The apparatus according to claim 58, wherein the processing unit is further configured to:
send second indication information to the first communication device, wherein the second indication information comprises the first concentration and/or the second concentration.

62. The apparatus according to any one of claims 55 to 61, wherein when performing the determining the first concentration of the first gas based on the receive power and the transmit power of the first communication signal, the processing unit is configured to:
determine the first concentration of the first gas based on the transmit power of the first communication signal, the receive power of the first communication signal, a distance between the second communication device and the first communication device, and an absorption coefficient of the first gas at the frequency of $f_1$.

63. The apparatus according to claim 62, wherein the first concentration satisfies:

$$M_A = \frac{1}{L \cdot \sigma(f_1)} \ln \frac{P_{A0}(f_1)}{P_A(f_1)},$$

wherein $M_A$ represents the first concentration, $\sigma(f_1)$ represents the absorption coefficient of the first gas at the frequency of $f_1$, $L$ represents the distance between the second communication device and the first communication device, $P_{A0}(f_1)$ represents the transmit power of the first communication signal, and $P_A(f_1)$ represents the receive power of the first communication signal.

64. The apparatus according to any one of claims 58 to 61, wherein when performing the determining the second concentration of the first gas based on the transmit power and the receive power of the first communication signal and the transmit power and the receive power of the second communication signal, the processing unit is configured to:
determine the second concentration of the first gas on a communication link between the first communication device and the first communication device based on the transmit power of the first communication signal, the receive power of the first communication signal, the transmit power of the second communication signal, the receive power of the second communication signal, a distance between the second communication device and the first communication device, an absorption coefficient of the first gas at the frequency of $f_1$, and an absorption coefficient of the first gas at a frequency of $f_2$.

65. The apparatus according to claim 64, wherein the second concentration satisfies:

$$M_{AB} = \frac{1}{\Delta L[\sigma(f_1) - \sigma(f_2)]} \ln \frac{P_B(f_2) \cdot P_{A0}(f_1)}{P_A(f_1) \cdot P_{B0}(f_2)},$$

wherein $M_{AB}$ represents the second concentration, $\sigma(f_1)$ represents the absorption coefficient of the first gas at the frequency of $f_1$, $\sigma(f_2)$ represents the absorption coefficient of the first gas at the frequency of $f_2$, $L$ represents the distance between the second communication device and the first communication device, $P_{A0}(f_1)$ represents the transmit power of the first communication signal, $P_A(f_1)$ represents the receive power of the first communication signal, $P_{B0}(f_1)$ represents the transmit power of the second communication signal, and $P_A(f_1)$ represents the receive power of the second communication signal.

66. The apparatus according to any one of claims 55 to 65, wherein both $f_1$ and $f_2$ belong to a licensed frequency band, or $f_1$ belongs to a licensed frequency band, and $f_2$ belongs to an unlicensed frequency band.

67. A communication apparatus, wherein the communication apparatus comprises a processor and a storage medium, the storage medium stores instructions, and when the instructions are run by the processor, the method according to any one of claims 1 to 12 is implemented, or the method according to any one of claims 13 to 21 is implemented, or the method according to any one of claims 22 to 33 is implemented.

68. A computer-readable storage medium, wherein the computer-readable storage medium comprises instructions, and when the instructions are run by a processor, the method according to any one of claims 1 to 12 is implemented, or the method according to any one of claims 13 to 21 is implemented, or the method according to any one of claims 22 to 33 is implemented.

69. A computer program product, wherein the computer program product comprises instructions, and when the instructions are run by a processor, the method according to any one of claims 1 to 12 is implemented, or the method according to any one of claims 13 to 21 is implemented, or the method according to any one of claims 22 to 33 is implemented.

FIG. 1

| |
|---|
| A first communication device sends a first communication signal to a second communication device | S201 |
| The first communication device receives a first echo signal of the first communication signal | S202 |
| The first communication device determines a first concentration of a first gas based on transmit power of the first communication signal and receive power of the first echo signal | S203 |
| The first communication device determines, based on the first concentration, whether to send a second communication signal to the second communication device | S204 |

FIG. 2

```
┌─────────────────┐                              ┌─────────────────┐
│     First       │                              │    Second       │
│ communication   │                              │ communication   │
│    device       │                              │    device       │
└─────────────────┘                              └─────────────────┘
```

S301: Send a first communication signal
to the second communication device

S302: Determine a first concentration
of a first gas based on receive power
and transmit power of the first
communication signal

S303: Send first indication information
to the first communication device based
on the first concentration; or send first
indication information to the first
communication device

S304: Determine, based on the
first indication information from
the second communication
device, whether to send a second
communication signal to the
second communication device

FIG. 3

```
┌─────────────────────────────────┐
│                                 │
│   ┌──────────────────┐   401    │
│   │                  │          │
│   │  Transceiver unit│          │
│   │                  │          │
│   └──────────────────┘          │
│            │                    │
│            │                    │
│   ┌──────────────────┐   402    │
│   │                  │          │
│   │  Processing unit │          │
│   │                  │          │
│   └──────────────────┘          │
│                                 │
│     Communication apparatus     │
└─────────────────────────────────┘
```

FIG. 4

FIG. 5

FIG. 6

FIG. 7

## INTERNATIONAL SEARCH REPORT

| | International application No. |
|---|---|
| | **PCT/CN2022/129033** |

**A. CLASSIFICATION OF SUBJECT MATTER**

H04W24/08(2009.01)i; H04W24/02(2009.01)i; H04W4/50(2018.01)i

According to International Patent Classification (IPC) or to both national classification and IPC

**B. FIELDS SEARCHED**

Minimum documentation searched (classification system followed by classification symbols)

IPC: H04W

Documentation searched other than minimum documentation to the extent that such documents are included in the fields searched

Electronic data base consulted during the international search (name of data base and, where practicable, search terms used)

CNTXT, ENTXTC, ENTXT, DWPI, CJFD, CNKI: 华为技术有限公司, 郑瑶瑶, 曾昆, 刘乔, 王光健, 感知, 通感, 浓度, 气体, 回波, 回音, 回程, 回馈, ISAC, sensing, gas+, echo, concentration, percept+, communication, feedback

**C. DOCUMENTS CONSIDERED TO BE RELEVANT**

| Category* | Citation of document, with indication, where appropriate, of the relevant passages | Relevant to claim No. |
|---|---|---|
| X | CN 104303049 A (GULFSTREAM AEROSPACE CORP.) 21 January 2015 (2015-01-21) description, paragraphs [0014]-[0035], and claims 1-20, and figures 1-4 | 1-3, 22-24, 34-36, 55-57, 67-69 |
| A | CN 107991269 A (LASER INSTITUTE, SHANDONG ACADEMY OF SCIENCES) 04 May 2018 (2018-05-04) entire document | 1-69 |
| A | CN 102809583 A (BEIJING WUZI UNIVERSITY) 05 December 2012 (2012-12-05) entire document | 1-69 |
| A | CN 112986903 A (THE CHINESE UNIVERSITY OF HONG KONG, SHENZHEN) 18 June 2021 (2021-06-18) entire document | 1-69 |
| A | CN 204314454 U (FLIR SYSTEMS, INC.) 06 May 2015 (2015-05-06) entire document | 1-69 |
| A | US 2013128271 A1 (THE AEROSPACE CORP.) 23 May 2013 (2013-05-23) entire document | 1-69 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **20 July 2023** | **26 July 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2022/129033**

### C. DOCUMENTS CONSIDERED TO BE RELEVANT

| Category* | Citation of document, with indication, where appropriate, of the relevant passages | Relevant to claim No. |
|---|---|---|
| A | US 2020191716 A1 (NEC CORP.) 18 June 2020 (2020-06-18) <br> entire document | 1-69 |
| A | WO 2021114055 A1 (HUAWEI TECHNOLOGIES CO., LTD.) 17 June 2021 (2021-06-17) <br> entire document | 1-69 |

Form PCT/ISA/210 (second sheet) (July 2022)

**INTERNATIONAL SEARCH REPORT**
Information on patent family members

| International application No. |
|---|
| **PCT/CN2022/129033** |

| Patent document cited in search report | | | Publication date (day/month/year) | Patent family member(s) | | | Publication date (day/month/year) |
|---|---|---|---|---|---|---|---|
| CN | 104303049 | A | 21 January 2015 | EP | 2798338 | A1 | 05 November 2014 |
| | | | | EP | 2798338 | B1 | 07 November 2018 |
| | | | | WO | 2013086158 | A1 | 13 June 2013 |
| | | | | US | 2013147493 | A1 | 13 June 2013 |
| | | | | US | 9448186 | B2 | 20 September 2016 |
| | | | | BR | 112014014003 | A2 | 04 July 2017 |
| | | | | BR | 112014014003 | B1 | 27 October 2020 |
| | | | | CA | 2868543 | A1 | 13 June 2013 |
| | | | | CA | 2868543 | C | 04 September 2018 |
| CN | 107991269 | A | 04 May 2018 | | None | | |
| CN | 102809583 | A | 05 December 2012 | CN | 102809583 | B | 08 April 2015 |
| CN | 112986903 | A | 18 June 2021 | CN | 112986903 | B | 15 October 2021 |
| CN | 204314454 | U | 06 May 2015 | WO | 2013147947 | A2 | 03 October 2013 |
| | | | | US | 2013169468 | A1 | 04 July 2013 |
| | | | | EP | 2798368 | A2 | 05 November 2014 |
| US | 2013128271 | A1 | 23 May 2013 | US | 8614794 | B2 | 24 December 2013 |
| US | 2020191716 | A1 | 18 June 2020 | WO | 2019003288 | A1 | 03 January 2019 |
| | | | | JPWO | 2019003288 | A1 | 26 March 2020 |
| | | | | JP | 6766963 | B2 | 14 October 2020 |
| | | | | US | 10976254 | B2 | 13 April 2021 |
| WO | 2021114055 | A1 | 17 June 2021 | CN | 114145049 | A | 04 March 2022 |

Form PCT/ISA/210 (patent family annex) (July 2022)